(19) **Europäisches Patentamt**

**European Patent Office**

**Office européen des brevets**

(11) **EP 1 079 893 B1**

(12) **EUROPEAN PATENT SPECIFICATION**

(45) Date of publication and mention
of the grant of the patent:
**03.08.2005 Bulletin 2005/31**

(51) Int Cl.⁷: **A61N 1/368**

(86) International application number:
**PCT/US1999/010142**

(21) Application number: **99921811.8**

(22) Date of filing: **07.05.1999**

(87) International publication number:
**WO 1999/058191 (18.11.1999 Gazette 1999/46)**

(54) **CARDIAC PACING USING ADJUSTABLE ATRIO-VENTRICULAR DELAYS**

HERZREIZUNG UNTER VERWENDUNG VON EINSTELLBAREN ATRIO-VENTRIKULÄREN VERZÖGERUNGSINTERVALLEN

STIMULATION CARDIAQUE UTILISANT DES RETARDS AURICULO-VENTRICULAIRES REGLABLES

(84) Designated Contracting States:
**AT BE CH DE FR GB IE IT LI NL**

(30) Priority: **08.05.1998 US 84704 P**
**08.05.1998 US 75278**

(43) Date of publication of application:
**07.03.2001 Bulletin 2001/10**

(73) Proprietor: **CARDIAC PACEMAKERS, INC.**
**St. Paul, Minnesota 55112-5798 (US)**

(72) Inventors:
• **DING, Jiang**
**Maplewood, MN 55109 (US)**
• **YU, Yinghong**
**Maplewood, MN 55109 (US)**
• **KRAMER, Andrew, P.**
**Stillwater, MN 55082 (US)**
• **SPINELLI, Julio**
**Shoreview, MN 55126 (US)**

(74) Representative:
**Beresford, Keith Denis Lewis et al**
**BERESFORD & Co.**
**16 High Holborn**
**London WC1V 6BX (GB)**

(56) References cited:
**EP-A- 0 474 958**          **US-A- 5 318 595**
**US-A- 5 334 222**          **US-A- 5 554 177**
**US-A- 5 690 689**          **US-A- 5 700 283**

## Description

The present invention relates generally to an apparatus for cardiac pacing and, in particular, to a pacing system providing adjustable atrio-ventricular time delays to improve different heart performance parameters.

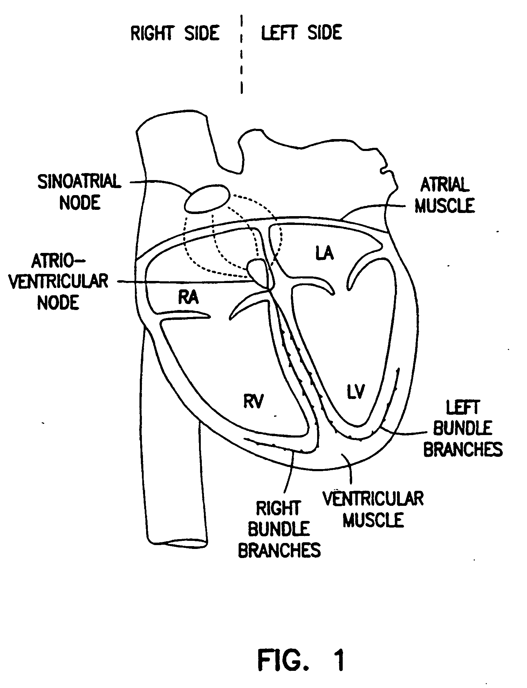

The heart is the center of the circulatory system. It is an organ which performs two major pumping functions and may be divided into right and left heart "pumps." The left heart pump draws oxygenated blood from the lungs and pumps it to the organs of the body. The right heart pump draws blood from the body organs and pumps it into the lungs. For a human heart, the right heart pump is on a patient's right side and the left heart pump is on the patient's left side. Figures in this document, such as FIG. 1, show a "top" view of the heart, which is the view that a physician observes during open heart surgery. Therefore, the left heart pump is on the right hand side of the FIG. 1 and the right heart pump is on the left hand side of FIG. 1. Each heart pump includes an upper chamber called an atrium and a lower chamber called a ventricle. The left heart pump therefore contains a left atrium (LA) and a left ventricle (LV), separated by a valve called the mitral valve. The right heart pump contains a right atrium (RA) and a right ventricle (RV), separated by a valve called the tricuspid valve.

The blood flows in the circulatory system in the following path: from the peripheral venous system (blood which has transferred through the body organs) to the RA, from the RA to the RV through the tricuspid valve, from RV to the pulmonary artery through the pulmonary valve, to the lungs. Oxygenated blood from the lungs is drawn from the pulmonary vein to the LA, from the LA to the LV through the mitral valve, and finally, from the LV to the peripheral arterial system (transferring blood to the organs of the body) through the aortic valve.

Normally, the heart pumps operate in synchrony and ensure the proper pumping action to provide oxygenated blood from the lungs to the organs of the body. A normal heart provides this synchrony by a complex conduction system which propagates electrical pulses to the heart muscle tissue to perform the necessary atrial and ventricular contractions. A heartbeat is the result of a regular train of electrical pulses to the proper portions of the heart to provide rhythmic heart pumping. The heart muscle provides pumping by the contraction of muscle tissue upon receipt of an electrical signal, and the pumping action is made possible through a system of heart valves which enable blood flow in a single direction. Thus, the heart includes a complex electrical and mechanical network.

To pump blood through the circulatory system, a beating heart performs a cardiac cycle. A cardiac cycle consists of a systolic phase and a diastolic phase. During systole, the ventricular muscle cells contract to pump blood through both the pulmonary circulation and the systemic circulation. During diastole, the ventricular muscle cells relax, which causes pressure in the ventricles to fall below that in the atria, and the ventricles begin to be refilled with blood.

In normal condition, the cardiac pumping is highly efficient. One aspect of this high efficiency is due to sequential atrio-ventricular contraction. Near the end of diastole, the atria contract, causing an extra amount of blood to be forced into the ventricles. Thus, the ventricles have more blood (preload) to pump out during next systole. Another aspect of this high efficiency in blood pumping is contributed from a network or fast ventricular conduction system. As shown in FIG. 1, the system includes right and left bundle branches of conductive tissues that extend from the Bundle of His and the massive network of fast conducting Purkinje fibers that cover most of the endocardial surface of the ventricles. Electrical signals coming from the atrium are relayed to the Purkinje fibers through the bundle branches, and to the different regions of the ventricles by the Purkinje fiber network. Therefore the entire ventricular muscle cells can contract synchronously during systole. This synchronized contraction enhances the strength of the pumping power.

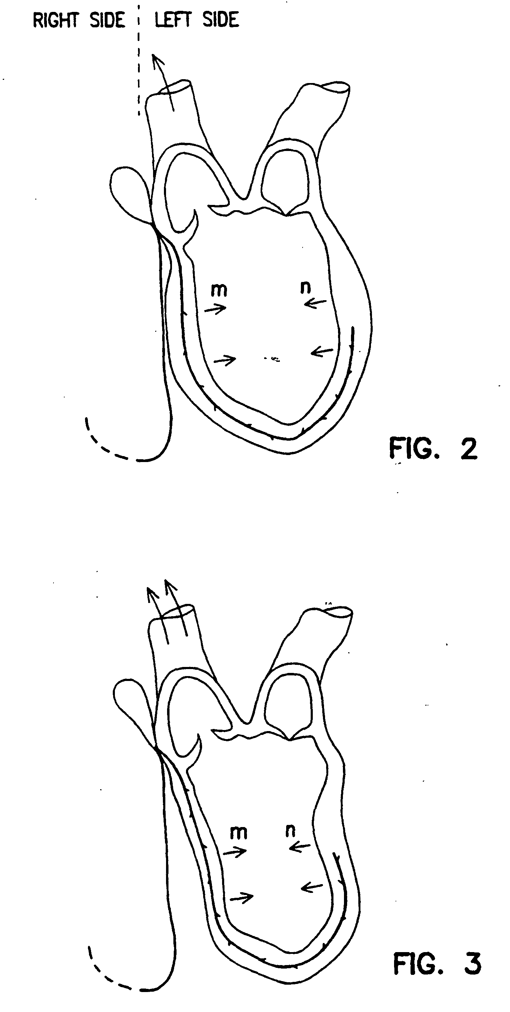

To assess the cardiac function, it is important to examine the LV systolic performance which directly determines the ability of the heart to pump blood through the systemic circulation. There are multiple ways to assess the performance of the heart. One way is to examine how well the LV contracts in order to determine the effectiveness of the LV as a pump. As can be seen from FIG. 2, the LV starts to contract after an electrical signal propagating down the left bundle branches stimulates muscle cells of septal wall M and lateral wall N. In FIG. 3, the walls M and N are contracting such that they are forced towards each other to pump blood out of the ventricle. One measure of LV contraction effectiveness is called "contractility." Left ventricular contractility is a measure of overall strength of the contracting power of the LV muscle cells. It is a function of the health of the LV muscle tissue and the coordination of the contractions of the entire LV, including walls M and N. Such coordination depends on the health of the left bundle branches and on the health of the fast conducting Purkinje fiber network. LV contractility is estimated by measuring the peak positive rate of change of the LV pressure during systole. In mathematical terms, this is the maximum positive derivative of the LV pressure, which is denoted by the term "LV +dp/dt".

LV systolic performance is also measured by stroke volume, which is the volume of blood pumped out of the LV per systole. Stroke volume can be estimated by measuring aortic pulse pressure (PP).

Cardiac muscle cells need to be electrically excited before they can have a mechanical contraction. During the excitation (depolarization), electrical signals will be generated and they can be recorded both intracardially and extracardially. The recorded signals are generally called electrocardiogram (ECG). An ECG recorded intracardially is also called an electrogram, which is recorded from an electrode placed endocardially or epicardially in an atrium or a ventricle. An ECG recorded extracardially is often called surface ECG, because it is usually recorded from two or more electrodes attached to the skin of the body. A complete surface ECG recording is from 12-lead configuration.

The features in ECG are labeled according to the origin of the electrical activity. The signals corresponding to intrinsic depolarization in an atrium and a ventricle are called P-wave and QRS complex, respectively. The QRS complex itself consists of a Q-wave, a R-wave, and a S-wave. The time interval from P-wave to R-wave is called PR interval. It is a measure of the delay between the electrical excitation in the atrium and in the ventricle.

Several disorders of the heart have been studied which prevent the heart from operating normally. One such disorder is from degeneration of the LV conduction system, which blocks the propagation of electric signals through some or all of the fast conducting Purkinje fiber network. Portions of the LV that do not receive exciting signals through the fast conducting Purkinje fiber network can only be excited through muscle tissue conduction, which is slow and in sequential manner. As a result, the contraction of these portions of the LV occurs in stages, rather than synchronously. For example, if the wall N is affected by the conduction disorder, then it contracts later than the wall M which is activated through normal conduction. Such asynchronous contraction of the LV walls degrades the contractility (pumping power) of the LV and reduces the LV+dp/dt (maximum positive derivative of the LV pressure) as well.

Another disorder of the heart is when blood in the LV flows back into the LA, resulting in reduced stroke volume and cardiac output. This disorder is called mitral regurgitation and can be caused by an insufficiency of the mitral valve, a dialated heart chamber, or an abnormal relationship between LV pressure and LA pressure. The amount of the back flow is a complex function of the condition of the mitral valve, the pressure in the LV and in the LA, and the rate of blood flow through the left heart pump.

These disorders may be found separately or in combination in patients. For example, both disorders are found in patients exhibiting congestive heart failure (CHF). Congestive heart failure (CHF) is a disorder of the cardiovascular system. Generally, CHF refers to a cardiovascular condition in which abnormal circulatory congestion exists as a result of heart failure. Circulatory congestion is a state in which there is an increase in blood volume in the heart but a decrease in the stroke volume. Reduced cardiac output can be due to several disorders, including mitral regurgitation (a back flow of blood from the LV to the LA) and intrinsic ventricular conduction disorder (asynchronous contraction of the ventricular muscle cells), which are the two common abnormalities among CHF patients.

Patients having cardiac disorders may receive benefits from cardiac pacing. For example, a pacing system may offer a pacing which improves LV contractility, (positive LV pressure change during systole), or stroke volume (aortic pulse pressure), however, known systems require complicated measurements and fail to provide automatic optimization of these cardiac performance parameters.

Furthermore, the measurements are patient-specific and require substantial monitoring and calibration for operation. Therefore, there is a need in the art for a system which may be easily adapted for optimizing various cardiac parameters, including, but not limited to, LV contractility, (peak positive LV pressure change during systole. LV+dp/dt), and cardiac stroke volume (pulse pressure). The system should be easy to program and operate using straightforward patient-specific measurements.

US-A-5,690,689 describes an implantable pacemaker according to the preamble of claim 1 in which AV intervals are automatically adjusted to be a function of the natural conduction time sensed by the pacemaker, where the natural conduction time is the time between atrial activity, such as a P-wave, and the subsequent ventricular activity, such as an R-wave.

Aspects of the present invention are set out the appended claims.

## Brief Description of the Drawings

FIG. 1 is a.diagram of a heart showing the chambers and the nervous conduction system.

FIG. 2 is a diagram of a ventricle beginning contraction.

FIG. 3 is a diagram of a contracted ventricle.

FIG. 4A is a graph of left ventricle intrinsic pressure as a function of time as referenced to an intrinsic P-wave event.

FIG. 4B is a graph of left ventricle intrinsic electrogram as a function of time as referenced to an intrinsic P-wave event.

FIG. 4C is a timing diagram showing a marker of an intrinsic P-wave and the marker of a ventricular pacing pulse that is optimally timed for maximum LV contractility as referenced to a paced P-wave event;

FIG. 4D is a graph of left atrial intrinsic pressure as a function of time as referenced to an intrinsic P-wave event.

FIG. 4E is a timing diagram showing a marker of an intrinsic P-wave and the marker of a ventricular pacing pulse that is optimally timed for maximum stroke volume as referenced to a paced P-wave event.

FIG. 5A is a flow diagram for detection of a Q* event.

FIG. 5B is one embodiment of an implantable medical device system implanted into a heart of a patient from which portions have been removed to show detail;

FIG. 5C is a block diagram of an implantable rhythm management device according to one embodiment of the present invention;

FIG. 5D is one embodiment of an implantable medical device system implanted into a heart of a patient from which portions have been removed to show detail;

FIG. 5E is one embodiment of an implantable medical device system implanted into a heart of a patient from which portions have been removed to show detail;

FIG. 5F is one embodiment of an electrocardiogram showing cardiac action currents of human subjects;

FIG. 6 is one embodiment of a schematic of an electrocardiogram showing cardiac action currents;

FIG. 7 is a flow diagram illustrating one embodiment of the present invention;

FIG. 8A, 8B, 8C and 8D are plots showing embodiments of correlations between sensed cardiac time intervals;

FIG. 9 is a plot showing an embodiment of optimal AV time delay intervals as a function of sensed cardiac time intervals;

FIG. 10 is a flow diagram illustrating one embodiment of the present invention;

FIGS. 11A and 11B are plots showing embodiments of optimal AV time delay intervals as a function of sensed cardiac time intervals; and

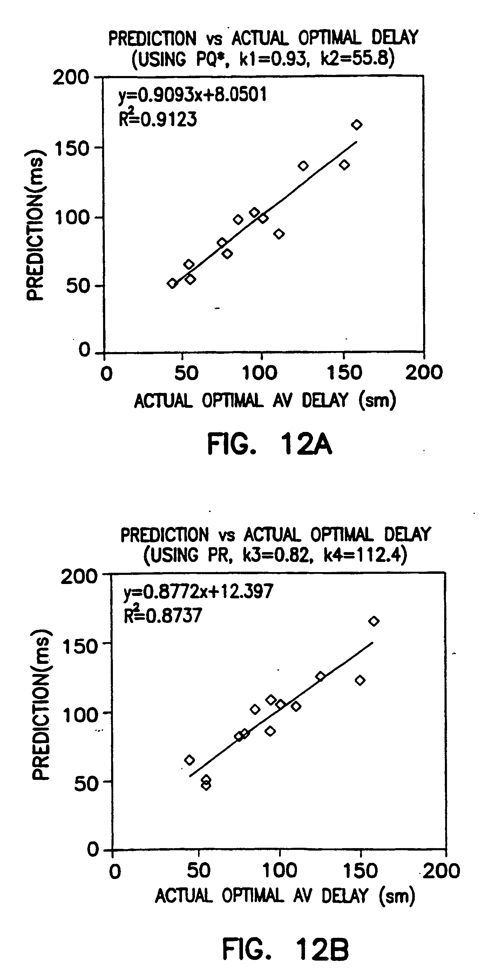

FIGS. 12A and 12B are plots showing embodiments of estimated optimal AV time delay intervals as a function of actual optimal AV time delay intervals.

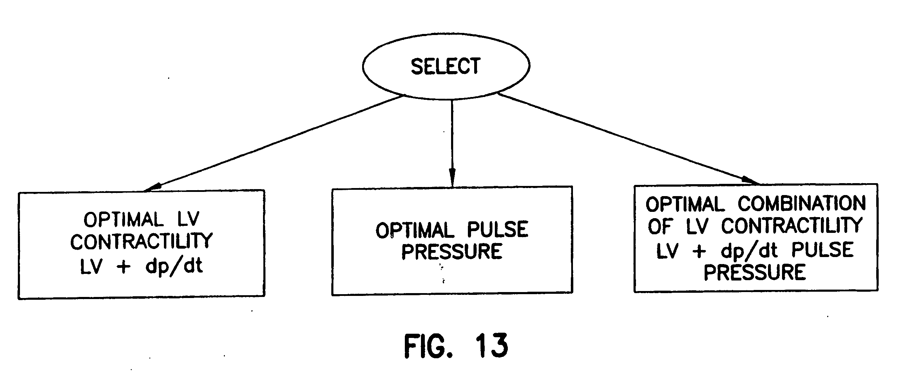

FIG. 13 shows the selection between optimal hemodynamic cardiac parameters according to one embodiment of the present system.

## Summary of the Invention

This patent application describes multiple ways to provide optimized timing for ventricular pacing by determining certain intrinsic electrical or mechanical events in the atria or ventricles that have a predictable timing relationship to the delivery of optimally timed ventricular pacing that maximizes ventricular performance. This relationship allows prediction of an atrio-ventricular delay used in delivery of a ventricular pacing pulse relative to a sensed electrical P-wave of the atrium to establish the optimal pacing timing. Also provided are embodiments for measuring these events and deriving the timing relationship above. Those skilled in the art will understand upon reading the description that other events may be used without departing from the present invention.

In several embodiments, these measurements are used to optimize ventricular contractility as measured by maximum rate of pressure change during systole. In other embodiments, these measurements are used to optimize stroke volume as measured by aortic pulse pressure. In other embodiments, a compromise timing of pacing is available to provide nearly optimal improvements in both peak positive pressure change during systole and aortic pulse pressure. In one embodiment, this pacing is provided by adjusting the atrio-ventricular delay time interval, which is the time interval after a sensed P-wave, to deliver a pacing pulse to achieve the desired cardiac parameter optimization.

This summary of the invention is intended not to limit the claimed subject matter, and the scope of the invention is defined by attached claims.

## Detailed Description

In the following detailed description, reference is made to the accompanying drawings which form a part hereof and in which is shown by way of illustration specific embodiments in which the invention can be practiced. These embodiments are described in sufficient detail to enable those skilled in the art to practice and use the invention, and it is to be understood that other embodiments may be utilized and that electrical, logical, and structural changes may be made without departing from the scope of the present invention. The following detailed description is, therefore, not to be taken in a limiting sense and the scope of the present invention is defined by the appended claims.

Some of the embodiments illustrated herein are demonstrated in an implantable cardiac pacemaker, which may include numerous pacing modes known in the art. However, these embodiments are illustrative of some of the applications of the present system, and are not intended in an exhaustive or exclusive sense. For example, the present system is suitable for implementation in a variety of implantable and external devices.

The present system provides a means for optimizing cardiac systolic function based on different cardiac performance measurements. The present disclosure provides a number of embodiments useful for, among other things, optimizing cardiac pumping strength and stroke volume. The concepts described herein may be used in a variety of applications which will be readily appreciated by those skilled in the art upon reading and understanding this description. The cardiac performance measurements expressly provided herein include contractility, peak positive ventricular pressure change, stroke volume, and pulse pressure. Other cardiac performance may be maximized using the teachings provided herein, and therefore, the express teachings of this disclosure are not intended in an exclusive or limiting sense. These concepts are expressly described in terms of the left ventricle, however, applications to other chambers of the heart, including the right ventricle, may be readily appreciated by those skilled in the art without departing from the present invention.

The inventors of this subject matter performed numerous tests and experiments to develop a pacing system which may be used to treat cardiac disorders. The system includes method and apparatus which are useful for providing optimization of different cardiac performance parameters, including, but not limited to, ventricular contractility, maximum rate of pressure change during systole, stroke volume, and pulse pressure. The embodiments provided herein use right atrial (RA) sensing events to time the pacing of the left ventricle (LV), right ventricle (RV), or both (BV) to optimize cardiac performance parameters. However, it is understood that these teachings are applicable to other pacing configurations. The teachings herein provide, among other things, optimal pacing which is selectable for treating different cardiac disorders. The disorders include, but are not limited to, congestive heart failure (CHF), mitral regurgitation, and ventricular conduction disorder. The optimal pacing taught herein includes embodiments which do not use patient-specific measurements of hemodynamic parameters, such as pressure, blood flow, or measurements not typically provided by implantable pacing devices, and the system is capable of automatic adjustment to meet the needs of a particular patient.

## AVD Time Intervals

Implantable rhythm management devices such as pacemakers, are useful for treating patients with abnormal cardiac functions. One pacing therapy is called DDD pacing mode. In DDD pacing mode, pacing electrodes are placed in the atrium (for example, the RA) and one or both of the ventricles. These electrodes are also used to sense electric signals from the atrium and the ventricle(s). If the device senses a signal in the atrium, it will inhibit the delivery of a pacing pulse to the atrium, otherwise it will pace the atrium after the end of a predetermined time period. Whenever the device senses or paces the atrium, it generates an event marker and at the same time starts an atrio-ventricular delay (AVD) time interval. At the end of this delay interval, the device will pace the ventricle(s) if no signals from the ventricle(s) are sensed by the device. Systems which provide ventricular pacing signals relative to the P-wave of an electrocardiogram signal refer to atrio-ventricular time delay interval (AVD time interval) as the time delay from the sensed P-wave to the delivery of the ventricular pacing signal. In patients exhibiting ventricular conduction disorder, such as the CHF condition, therapy using an AVD time interval which is shorter than the PR time interval may provide improved contractility because patients with degeneration of their LV conduction system require pacing of the affected parts of the LV (for example, the lateral wall N) early enough so that the contraction may be in phase with other parts of the LV that are excited by intrinsic conduction (for example wall M). Properly timed ventricular pacing can make both walls M and N contract in phase for increased contractility.

Patients with decreased stroke volume benefit from a shorter AVD time interval to decrease the mitral regurgitation effects and increase aortic pulse pressure. In addition, for congestive heart failure (CHF) patients, their PR interval may be prolonged which reduces the AV synchrony to some extent. Such a reduction in AV synchrony may further increase mitral regurgitation, and reduce the effect of preload of the LV. Use of a shorter AVD time interval increases pulse pressure by forcing the contraction of the LV into an earlier period, thus reducing the effects of mitral regurgitation.

## Optimization of Cardiac Ventricle Contractility and Maximum Left Ventricle Pressure Change during Systole

Left ventricle contractility (pumping power) and peak positive rate of change of left ventricle pressure during systole (abbreviated as "LV+dp/dt") are related cardiac performance parameters. For instance, increases in LV contractility are observed in measurements as increases in left ventricle pressure change during systole.

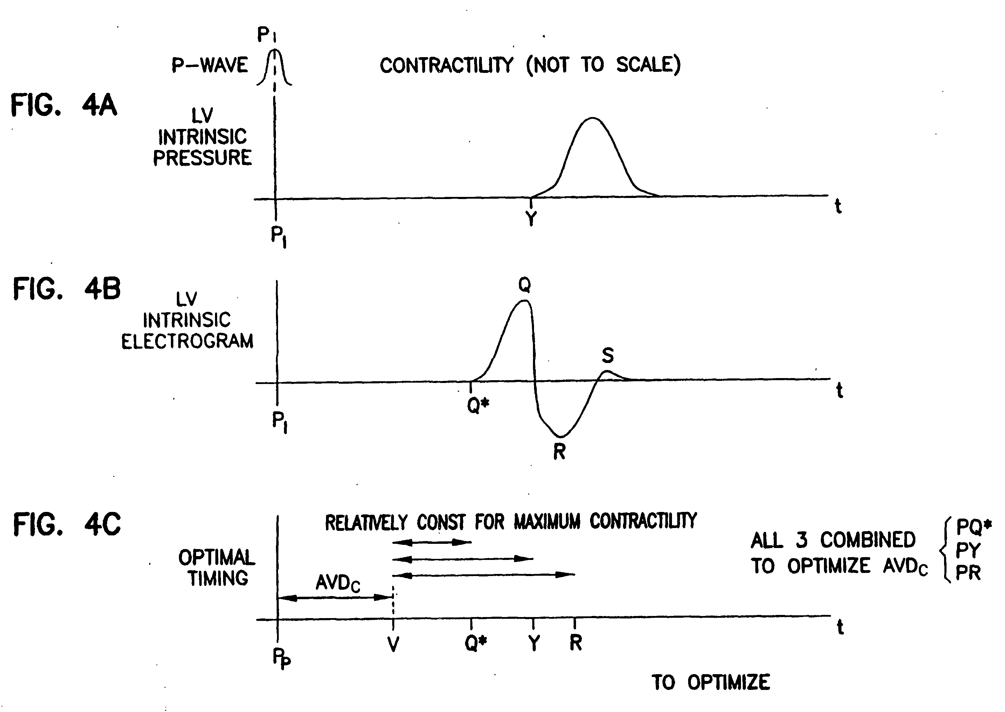

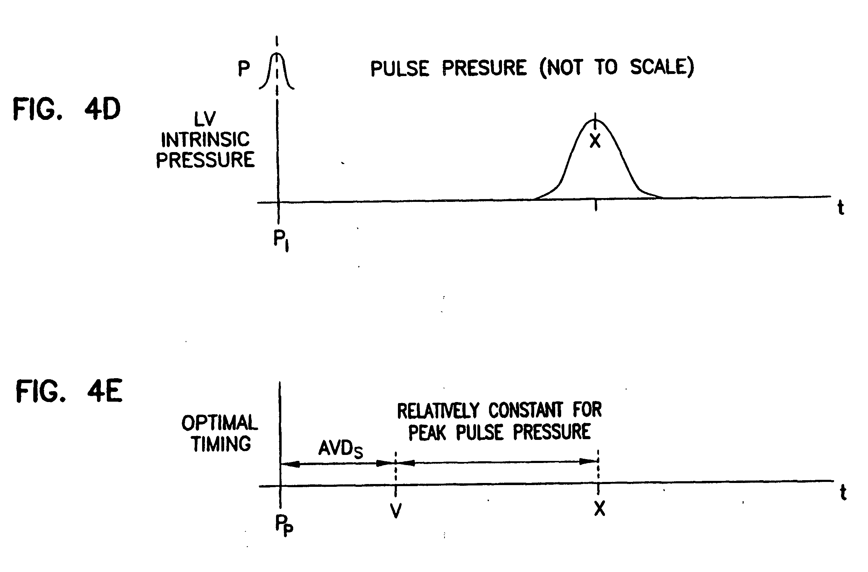

FIG. 4A shows an intrinsic or unpaced left ventricle pressure curve following a P-wave. The Y event is the onset of intrinsic LV pressure increase. FIG. 4B shows an intrinsic left ventricular electrogram which is a QRS complex following a P-wave. $Q^*$ is an electrical signal which occurs at the beginning of a QRS complex. R is the largest peak of the QRS complex. In FIG. 4B, the $Q^*$ event leads the Y event of FIG. 4A. FIG. 4C shows a timing diagram under an optimally paced condition in which the LV contractility is maximized. The $AVD_c$ time interval is equal to the time between the P-wave marker and the ventricular pacing marker V and that pacing provides maximum LV contractility. It is therefore called an optimal atrio-ventricular delay for contractility. It is noted that in the FIG. 4C the $P_p$ marker is from a paced condition, as opposed to the $P_I$ markers in FIGS. 4A and 4B, which arise from intrinsic heart activity. Therefore $P_p$ occurs at a different time than $P_I$. Additionally, the diagrams are not to scale.

In their experimentation, the inventors learned that when pacing for maximum contractility the $Q^*$, Y, and R events had a relatively predictable timing relationship with respect to the V pacing signal that is optimally timed by $AVD_c$. Furthermore, the inventors learned that linear models could be created which map the $PQ^*$ interval (the time difference between a P event and a $Q^*$ event) to an optimal atrio-ventricular delay for maximum contractility, $AVD_c$. Additionally, linear mappings are possible for PY and PR to $AVD_c$, however, each mapping may result in different coefficients.

In one embodiment, an intrinsic PQ* time interval is measured for a patient. This is the time interval between the P-wave and a Q* event when no pacing signal is applied. After the PQ* time interval is recorded and averaged, then a pacing signal is applied with varying atrio-ventricular delays while monitoring LV+dp/dt (peak positive left ventricular pressure change). Then the atrio-ventricular delay which produced the maximum LV+dp/dt (optimal contractility) is determined and named as $AVD_c$, and is paired with that patient's PQ* time interval. The PQ*, $AVD_c$ pairs are generated for a number of other patients and the data are plotted. In one embodiment, a linear regression method is applied to determine a straight line approximation for $AVD_c$ as a function of PQ*. The equation is: $AVD_c$ = K1(PQ*) - K2. A programmable device which measures the intrinsic PQ* interval can estimate $AVD_c$ using this equation. Therefore, once K1 and K2 are determined, the calibration of the device is complete. This means that subsequent patients may have optimal contractility pacing without requiring the pressure measurements and additional calibration stages. As described below, the same procedures may be used with PY or PR, however, as stated before, the coefficients may be different.

This means that, if PQ* is measured, then a patient may receive optimal contractility pacing of the left ventricle using measurements of the P-wave and of Q*. In the case where PY is used instead of PQ*, then the measurements will be of the P-wave and of the Y event, which is the onset of pressure increase in the left ventricular contraction. If the PR interval is used, then the measurements will be the P-wave and the R-wave of the QRS complex.

Therefore, given a patient's intrinsic PQ* or PY or PR time interval and the respective mapping, an $AVD_c$ is calculated. This $AVD_c$ is an approximation of the actual $AVD_c$ using the mapping method.

It is noted that any event which is relatively constant with respect to the optimally timed V pacing signal (pacing using $AVD_c$) may be used as a predictable event for use in the present system. In one embodiment, an event which is relatively constant is one which has a deviation between the lesser of 20 ms or 25 percent of the population mean. Therefore, other embodiments incorporating events not expressly mentioned herein may be used without departing from the present system.

P-Wave Signal

When the electronic P-wave signal is used as a reference for any of the embodiments, the P-wave signal is detectable using devices including, but not limited to, catheters or external probes to create electrocardiograms. In one embodiment, the P-wave is sensed from the right atrium and used as a reference for the time interval measurements and pacing delivery. In some cases where a patient's atrium is paced then the P-wave pacing marker is used instead of the intrinsic P-wave.

PQ* Measurement and Mapping

As stated above, the inventors determined some "events" would have a predictable relationship to the optimally timed ventricular pacing signal. The Q* event was defined as one candidate because it is relatively constant relative to the LV pacing mark, V, at optimal timing for maximum contractility. Q* is an electrical signal which occurs at the beginning of a QRS complex. Therefore, in one embodiment of the system, the time delay between the P-wave and the Q* event is used to provide the linear variable to calculate $AVD_c$. In this embodiment, the equation is: $AVD_c$ = K1 (PQ*) - K2.

Furthermore, the inventors of the present system realized that the PQ* interval provides a linear variable which may be used to estimate $AVD_c$ using a single calibration procedure for determining the constants K1 and K2. One type of calibration was discussed above, mapping $AVD_c$, PQ* pairs in a linear fashion to provide K1 and K2. The PQ* and $AVD_c$ information is then plotted on a two-dimensional chart and a linear regression method is performed to provide a best line fit through the sample point pairs. This linear fit provides the K1 and K2 coefficients.

In one study using 13 patients, an equation for $AVD_c$ was generated which provided K1 equal to 0.94 and K2 equal to 55.7 milliseconds. In this equation, PQ* is measured in milliseconds. This equation is expressed as: $AVD_c$ = 0.94 PQ* - 55.7 milliseconds. It is noted that the coefficients may vary and that estimated $AVD_c$ may depart from the actual optimum $AVD_c$ by approximately 20 percent and continue to provide near optimal performance within 80 percent of the maximum contractility. Furthermore, the coefficients may vary slightly depending on the number of samples taken in the calibration stage. Therefore, the coefficients provided herein may vary without departing from the present invention.

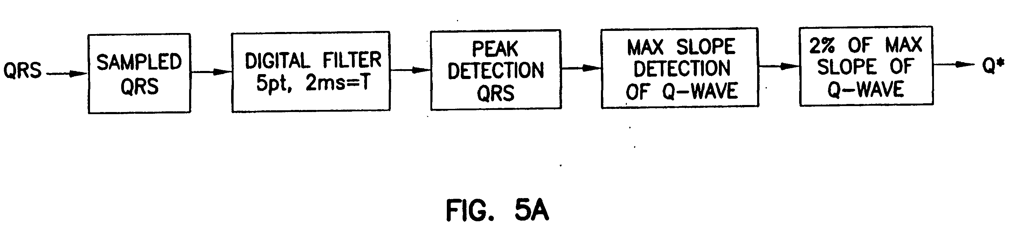

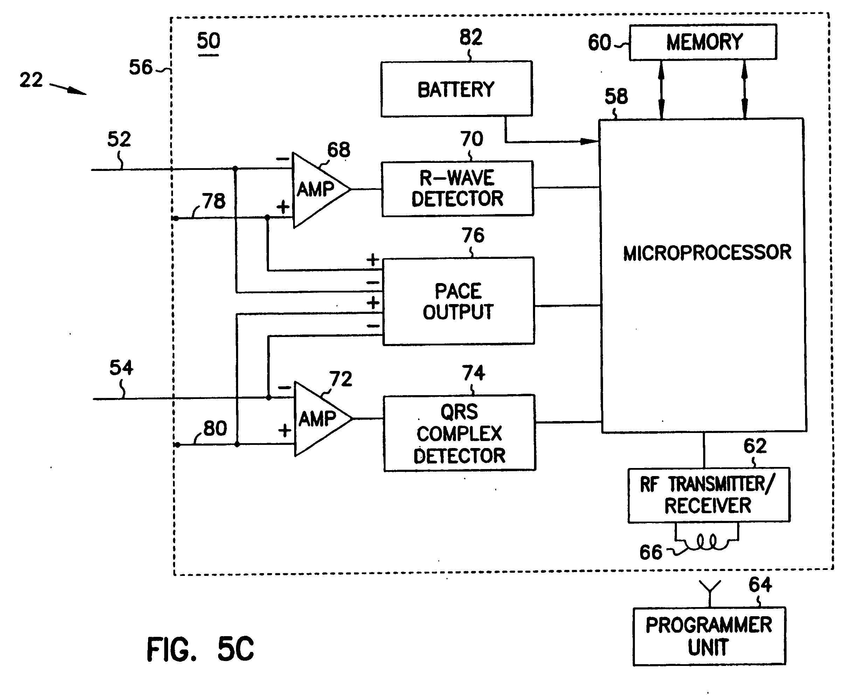

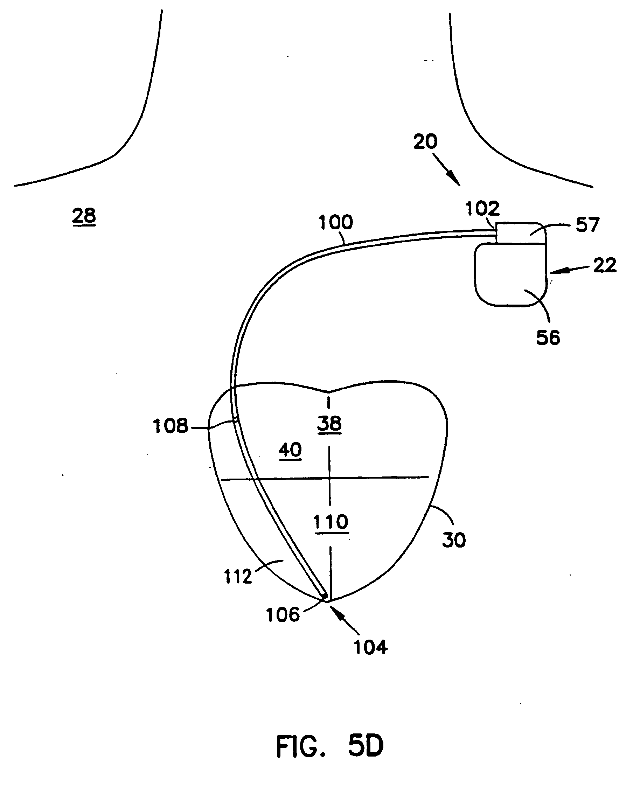

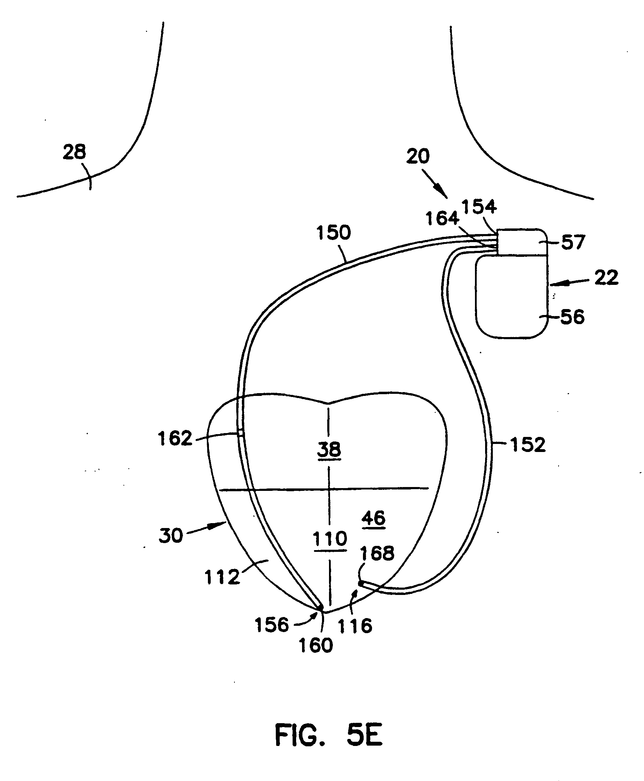

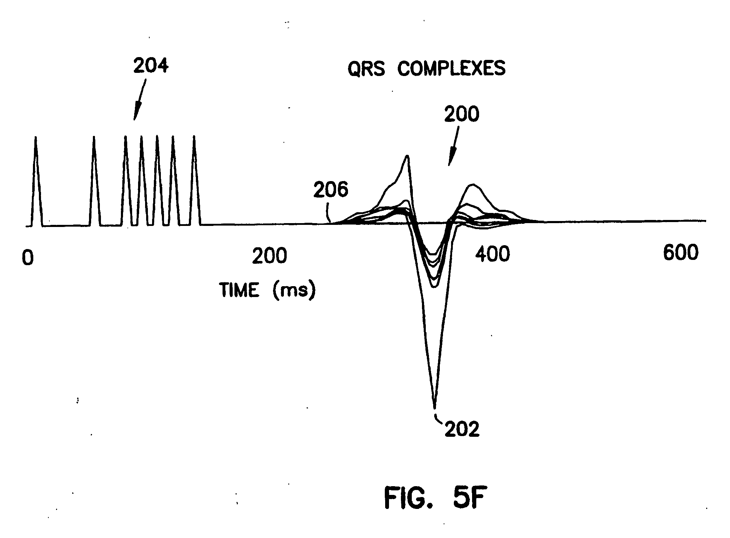

In one embodiment, the P-wave was detected using a threshold detector which indicated a P-wave at approximately 20 percent of the maximum P-wave amplitude in the right atrium. In one embodiment shown in FIG. 5A, the Q* event is determined by passing the QRS complex as sampled from the left ventricle through a 5 point low-pass digital filter having a sampling time of 2 milliseconds, detecting the Q portion of the wave, calculating a maximum absolute value of the slope for the Q-wave, and indicating a point on the filtered Q-wave where the absolute value of the slope equals 2% of the absolute value slope of the Q-wave. Those skilled in the art will readily recognize that other determination methods may be used for P and Q* which do not depart from the present system. Changes in the measurement techniques and slope criteria do not depart from the present system.

In another embodiment, the coefficient of PQ*, K1, is assumed to be unity, and the coefficient K2 amounts to an offset time delay from the PQ* interval to predict or estimate the optimal $AVD_c$. In this embodiment, PQ* and $AVD_c$ are sampled for a variety of patients at a variety of PQ* intervals and a variety of $AVD_c$ to generate a mean offset time delay K2 for a number of patients. In this embodiment, the equation is as follows: $AVD_c$ estimated = PQ* - Wa milliseconds. Using the previous data for the 13 patients, the equation is: $AVD_c$ estimated = PQ* - 67 milliseconds. This embodiment provides an easier calculation, since a subtraction is less processor intensive than multiplications using floating point numbers. However, some accuracy is lost for the approximation.

It is noted that the coefficients may vary and that the estimated $AVD_c$ may depart from the actual optimum $AVD_c$ by approximately 20 percent and continue to provide near optimal performance within 80 percent of the maximum contractility. Furthermore, the coefficients may vary slightly depending on the number of samples taken in the calibration stage. Therefore, the coefficients provided herein may vary without departing from the present invention.

Those skilled in the art will readily recognize that other methods may be employed to generate other fits to the data which do not depart from the scope of the present invention.

In one embodiment, the measurements of the P-wave and Q* are provided using an electrode implanted in the right atrium and an electrode implanted in the left ventricle. A programmable pulse generator is used to sense the P-wave and measure the time between occurrence of a sensed P-wave and a sensed Q* event. The Q* event is determined by electronics in the pulse generator which perform the required slope and comparison operations to determine Q*. After a PQ* time interval is determined, the $AVD_c$ is determined using any of the embodiments described herein and their equivalents. Once the $AVD_c$ is determined, it may be used in the next pacing interval to provide an optimized atrio-ventricular delay based on the PQ* time interval.

It is understood that the Q* event may be defined differently and provide substantially the same results with a different set of parameters, K1 and K2. Furthermore, any electrical signal event which bears a predictable relationship to the beginning of intrinsic LV electrogram signals may be used in place of Q*. For example, in one embodiment the beginning of the RV electrogram may be used in place of Q*. Or in another embodiment, the Q* may be measured by surface ECG as the onset of the signal averaged QRS complex. Furthermore, information from more than one lead may be used to more accurately determine Q*.

PR Measurement and Mapping

In another embodiment, the R-wave peak, which is the largest peak of the QRS complex of an intrinsic LV electrogram, is used since it has a predictable relationship to the delivery of optimally timed ventricular pacing for maximum contractility. In particular, the linear time relationship may be derived in terms of the PR interval for optimal atrio-ventricular delay for optimal left ventricular pressure change during systole. In this case, the equation is: $AVD_c$ = N1 PR - N2, where $AVD_c$ is for pacing the LV, and PR is the time interval from right atrial sensing marker to the largest peak of the QRS complex of intrinsic LV electrogram. In one embodiment, the N1 and N2 coefficients are determined by mapping the PR time interval to the optimal $AVD_c$ for a number of patients for optimal left ventricular pressure change during systole. In one study using 13 patients, the coefficient N1 is equal to 0.82 and the coefficient N2 is equal to 112 milliseconds. The equation for this calibration is: $AVD_c$ = 0.82 PR - 112 milliseconds. It is noted that the coefficients may vary and that the estimated $AVD_c$ may depart from the actual optimum $AVD_c$ by approximately 20 percent and continue to provide near optimal performance within 80 percent of the maximum contractility. Furthermore, the coefficients may vary slightly depending on the number of samples taken in the calibration stage. Therefore, the coefficients provided herein may vary without departing from the present invention.

In another embodiment, the N1 coefficient is assumed to be unity, and the PR, $AVD_c$ data pairs are averaged to provide a linear dependence with an offset equal to N2. This embodiment provides an easier calculation, since a subtraction is less processor intensive than multiplications using floating point numbers. However, some accuracy is lost for the approximation. For example, using data in the previous study: $AVD_c$ = PR - 159 milliseconds. In one embodiment, the R-wave signal is measured by detecting the largest peak of the QRS complex of the intrinsic LV electrogram. Therefore, electrical signals are used in this embodiment to provide the PR time interval, and therefore the optimal atrio-ventricular delay for optimal left ventricular pressure change during systole. The coefficients N 1 and N2 are provided in an initial calibration stage, which means that subsequent readings using this embodiment generate the optimal $AVD_c$ automatically upon detection of the PR time interval. Furthermore, the N1 and N2 variables may change in value without departing from the teachings provided herein.

Other features of the QRS complex may be used for measurement. As stated above, these events may be used as long as they have a predictable timing relationship to the delivered pacing for optimal contractility. It is noted that the coefficients may vary and that the estimated $AVD_c$ may depart from the actual optimum $AVD_c$ by approximately 20 percent and continue to provide near optimal performance within 80 percent of the maximum contractility. Furthermore, the coefficients may vary slightly depending on the number of samples taken in the calibration stage. Therefore, the coefficients provided herein may vary without departing from the present invention.

PY Measurements and Mappings

In another embodiment, a mechanical event is provided as a reference instead of an electrical event. In one embodiment, the mechanical event, Y is determined as the beginning of intrinsic LV pressure development. This means that a pressure transducer such as a micromonometer can provide instantaneous pressure data in the left ventricle. In this embodiment, the atrio-ventricular delay optimized for maximum left ventricular pressure change during systole is provided as: $AVD_c$ = M1 PY - M2. In one embodiment, a micromonometer is placed in the LV to measure left ventricular pressure change during systole. The PY time interval, which is the time interval from right atrial sensing of the P-wave to the beginning of the intrinsic LV pressure development, is mapped to recorded $AVD_c$ values for maximum left ventricular pressure change during systole. This mapping is plotted to perform a linear regression in order to determine the coefficients M1 and M2. In one study, M 1 is equal to 0.96 and M2 is equal to 139 milliseconds. Therefore, in this study, the $AVD_c$ = 0.96 PY - 139 milliseconds. It is noted that the coefficients may vary and that the estimated $AVD_c$ may depart from the actual optimum $AVD_c$ by approximately 20 percent and continue to provide near optimal performance within 80 percent of the maximum contractility. Furthermore, the coefficients may vary slightly depending on the number of samples taken in the calibration stage. Therefore, the coefficients provided herein may vary without departing from the present invention.

In another embodiment; the M1 coefficient is approximated as unity, and then the PY and $AVD_c$ pairs are used to determine a linearized mapping which amounts to: $AVD_c$ = PY - $N_a$, where $N_a$ is an averaged offset delay for the samples taken. In one embodiment, $AVD_c$ = PY - 150 milliseconds. This embodiment provides an easier calculation, since a subtraction is less processor intensive than multiplications using floating point numbers. However, some accuracy is lost for the approximation. Again, it is noted that the coefficients may vary and that the estimated $AVD_c$ may depart from the actual optimum $AVD_c$ by approximately 20 percent and continue to provide near optimal performance within 80 percent of the maximum contractility. Furthermore, the coefficients may vary slightly depending on the number of samples taken in the calibration stage. Therefore, the coefficients provided herein may vary without departing from the present invention.

Other mechanical events may be used as long as they are relatively predictable with respect to the Y event. The Y events may be selected from signals including, but not limited to, ventricular pressure, cardiac phonogram, cardiac acoustic signals (such as recorded from an accelerometer external to or inside an implantable device), Doppler recording of atrio-ventricular valve motion, and M-mode, 2D, or 3D echo imaging of ventricular wall motion.

**Stroke Volume Optimization Using Atrio-Ventricular Delay**

Stroke volume is related to pulse pressure. The inventors discovered that for maximum pulse pressure (stroke volume), there is a predictable timing relationship between an optimally delivered ventricular pulse V and the peak of left atrial systole, X. Therefore, the optimal atrio-ventricular delay for maximum pulse pressure, $AVD_s$, is determined by PX time interval measurements, as shown in FIG. 4E.

In one embodiment, stroke volume is optimized by determining the atrio-ventricular delay for maximum aortic pulse pressure, $AVD_s$. In one embodiment, the X event is measured by placing a pressure sensing catheter inside the LA. In another embodiment, the X event is detected by measuring the LV pressure, because the LA contraction is seen in the LV pressure curve by a pre-systolic component. The peak of the LA systole is considered the same as the pre-systolic pressure in the LV pressure curve. The time interval between P and the pre-systolic component of LV pressure provides a linear equation. Therefore, in order to generate the linear mapping of PX to $AVD_s$, a number of PX, $AVD_s$ pairs are generated by measuring maximum aortic pulse pressure for varying PX. The linear relationship is expressed by: $AVD_s$ = M3 PX - M4 milliseconds. In one embodiment, a calibration procedure was performed to generate a number of PX, $AVD_s$ pairs, which are mapped and a best line fit is performed to determine M3 and M4. In one embodiment, M1 is equal to 1.22 and M2 is equal to 132 milliseconds. Therefore, the $AVD_s$ relationship is: $AVD_s$ = 1.22 PX - 132 milliseconds. It is noted that the coefficients may vary and that the estimated $AVD_s$ may depart from the actual optimum $AVD_s$ by approximately 20 percent and continue to provide near optimal performance of the maximum stroke volume. Furthermore, the coefficients may vary slightly depending on the number of samples taken in the calibration stage. Therefore, the coefficients provided herein may vary without departing from the present invention.

In one embodiment, the P-wave event is measured using a threshold detection where the P-wave is determined to be 20 % of the maximum P-wave amplitude. Other detection methods for the P-wave may be used without departing from the present system. The X event may be determined by several ways, including but not limited to: locating the point of maximum atrial pressure, Doppler measurements, and S4 components of accelerator measurements.

Other embodiments using different values for M3 and M4 are possible without departing from the present system. Furthermore, other markers may be used which are directly related to the PX time interval provided in one embodiment.

It is noted that any event which is relatively constant with respect to the optimally timed V pacing signal (pacing using $AVD_s$) may be used as a predictable event for use in the present system. In one embodiment, an event which is relatively constant is one which has a deviation between the lesser of 20 ms or 25 percent of the population mean. Therefore, other embodiments incorporating events not expressly mentioned herein may be used without departing from the present system.

**Selection of Atrio-Ventricular Delay for Improved Contractility and Stroke Volume**

Depending on the condition of a heart and its disorders, optimal atrio-ventricular delay for maximum contractility may provide especially nonoptimal stroke volume. Likewise, optimal atrio-ventricular delay for maximized stroke volume may result in nonoptimal contractility. Therefore, in order to provide a compromised atrio-ventricular delay which provides an approximately optimal atrio-ventricular delay for both contractility and stroke volume, $AVD_{cs}$, it is desirable to have an atrio-ventricular delay which provides near optimal contractility and near optimal stroke volume. The inventors of the present system derived a relationship which provides a compromise between optimal contractility and optimal stroke volume. In one embodiment, the optimized atrio-ventricular delay, $AVD_{cs}$, is a linear relationship in the PR time interval, as follows: $AVD_{cs} = K3 \, PR_m - K4$ milliseconds. $PR_m$ is a time interval measured from a right atrial sensing marker, P, to a right ventricular sensing marker, $R_m$. In one embodiment, the compromised $AVD_{cs}$ is provided by determining $AVD_c$ and $AVD_s$ for a number of PR values and for a number of patients. Then a linear regression provides a best line fit for both contractility and stroke volume. In one embodiment, $AVD_{cs}$ equals $0.5 \, PR_m - 15$ milliseconds, where $AVD_{cs}$ is for pacing at least one ventricle, and where the time interval $PR_m$ is measured from a right atrial sensing marker, P, to a right ventricular sensing marker, $R_m$. In this embodiment, the resulting atrio-ventricular delay provides a left ventricular pressure change within 90% of the optimal left ventricular pressure change during systole. Furthermore, this embodiment provides an aortic pulse pressure which is within 80% of the optimal aortic pulse pressure. It is noted that the coefficients may vary and still provide a reasonable approximation of $AVD_{cs}$. For example, in one embodiment K3 may be in the range from 0.4 to 0.6 and K2 may be in the range from 0 to 30 ms. Therefore, the present system offers flexibility in the selection of coefficients, and those provided are demonstrative and not an exclusive set of coefficients.

In one embodiment, a left ventricular event is used to provide a time interval for calculation of $AVD_{cs}$. In one case the LV event is the LV R-wave. The LV R-wave marker signal may also be used as an event. It is noted that any event which is relatively constant with respect to the near optimally timed V pacing signal may be used as a predictable event for use in the present system. In one embodiment, an event which is relatively constant is one which has a deviation between the lesser of 20 ms or 25 percent of the population mean. Therefore, other embodiments incorporating events not expressly mentioned herein may be used without departing from the present system.

In one embodiment, the left ventricular R wave is used to develop a relationship between the PR interval (the time interval between a P event and an R event) and $AVD_{cs}$. For a particular patient, the intrinsic PR interval is measured. Additionally, a sweep of atrio-ventricular delays are applied to the pacing of the patient and LV+dp/dt and pulse pressure are measured for each different atrio-ventricular delay. The LV+dp/dt data is plotted against a normalized value of the atrio-ventricular delay. Additionally, the pulse pressure is also plotted against a normalized value of the atrio-ventricular delay. In one embodiment, the atrio-ventricular delay is divided by PR-30 ms to normalize the delay. The tests are performed for a number of additional patients and the normalized plots are mapped. Then an averaging of the various LV+dp/dt vs. normalized atrio-ventricular delay data is performed. An averaging of the pulse pressure data vs. normalized atrio-ventricular delay data is also performed. The atrio-ventricular delay (normalized value) at the LV+dp/dt curve peak is used as an optimal averaged atrio-ventricular delay. The peak of the pulse pressure curve is also determined. In one example, the optimal averaged normalized atrio-ventricular delays for both curves was determined to be approximately 0.50 times the normalized PR time interval, or 0.50(PR-30) milliseconds.

In one study data was taken using a series of intermittent pacing (5 pacing beats in every 15 sinus beats) from one of three sites (RV, LV, and BV) at one of five AV delays (equally spaced between 0 msec and PR-30 msec). Each pacing site/AV delay combination was repeated five times in random order. Pressure and electrogram data were recorded from the ventricles. LV+dp/dt and PP were measured from LV and aortic pressure recordings on a beat-by-beat basis. For each paced beat, values of the LV+dp/dt and PP were compared to a preceding 6-beats-averaged sinus baseline. Then the response to pacing configuration was averaged. However, other measurements may be taken to obtain the required information.

**Switchable Pacing Therapies**

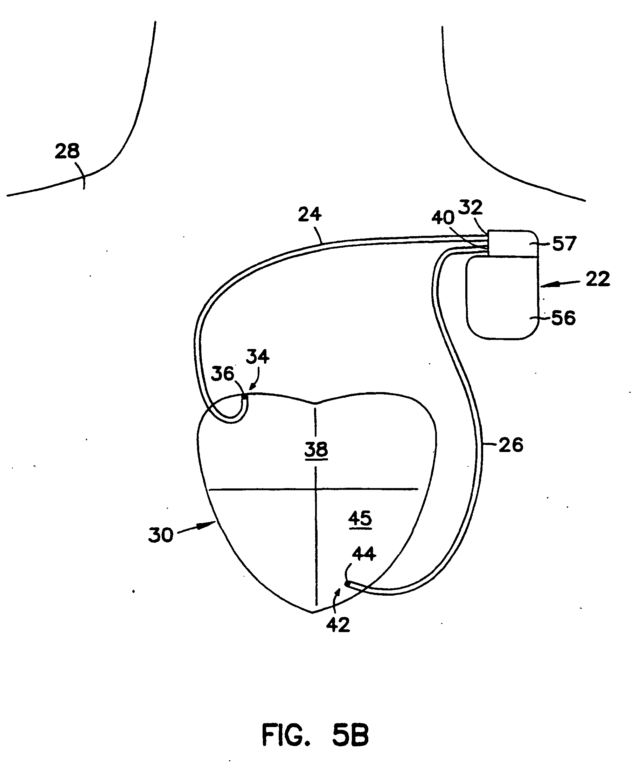

Anv of the teachings provided herein may be employed in a variety of cardiac devices, including implantable pacing devices, such as shown in FIGS 5B-5E. In one embodiment, an implantable device also includes means for changing the ventricular pacing to adjust for maximum contractility, maximum stroke volume or a compromise providing nearly optimal contractility and stroke volume. In such an embodiment, the pacing system contemplates the use of all of the different optimal atrio-ventricular delays to adjust the therapy to a cardiac patient. In one embodiment $AVD_{cs}$ is used as a default atrio-ventricular pacing delay, which may be maintained or modified at a later time depending on the therapy required. For example, in one embodiment of the system, the pacing initiates with an atrio-ventricular delay equal to $AVD_{cs}$. If at any time an optimal contractility is required, the atrio-ventricular pace delay is changed to $AVD_c$. Additionally, if at any time optimal stroke volume is required, the atrio-ventricular delay is changed to $AVD_s$. Other variations and combinations are possible without departing from the present invention. Furthermore, the switching of the pacing therapies may be provided by an external instruction, such as a programmer, or by an internally executing software for selecting the appropriate therapy. Other ways of switching between therapies may be encountered which do not depart from the present system.

**Experimental Findings**

Referring now to FIG. 5F, there is shown electrocardiogram traces of left ventricular QRS complexes 200 from patients having congestive heart failure (CHF). The QRS complexes 200 were recorded epicardially from the free wall regions of the patient's left ventricle. FIG. 5F shows the QRS complexes 200 having the peaks, or maximum deflection points, of the R points 202 aligned. The data shows a uniform pattern in electrical depolarization in the left ventricle of CHF patients, as the QRS complexes 200 have very similar durations. Sensed P-waves 204 from each patient are also displayed. As the data indicates, aligning the maximum deflection points of the R points 202 shows that the time interval between the atrial sensing or pacing marks of the P-waves 204 and the start 206 of the QRS complexes 200 varies from patient to patient. Because the R points 202 have been aligned, this necessarily means that the PR interval, the time interval between the atrial sensing or pacing mark of a sensed P-wave 204 and the maximum deflection point of a sensed R point 202, also varies from patient to patient.

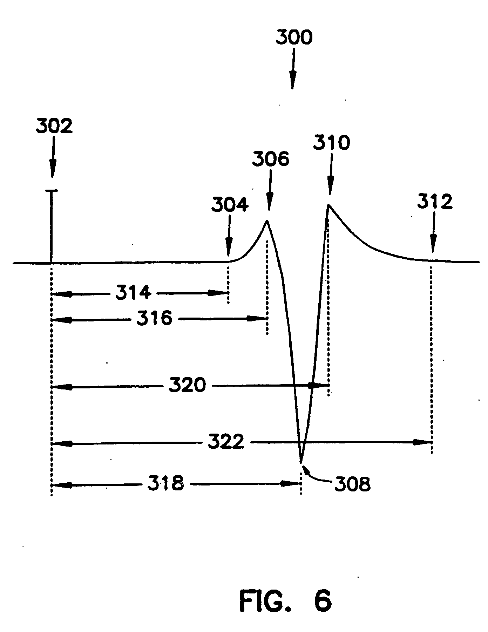

Referring now to FIG. 6, there is shown a schematic drawing of an electrocardiogram 300. The electrocardiogram 300 includes indications of cardiac events that occur during the normal cardiac cycle. The indications are shown in FIG. 6, where 302 represents the atrial sensing or pacing mark of a P-wave; 304 represents a Q* point, the beginning of the QRS complex; 306 represents the occurrence of a Q point, the maximum point before the beginning of the R point; 308 represents the occurrence of the R point, the maximum deflection point of the QRS complex in sinus rhythm; 310 represents the occurrence of the S point, the maximum point after the R point; and 312 represents an S* point, the end of the QRS complex. In one embodiment, these cardiac events displayed in electrocardiogram 300 are detected through the use of an implantable rhythm management device.

The electrocardiogram 300 also shows representations of a variety of time intervals between the cardiac events. A PQ*-time interval 314 is the time interval between a sensed P-wave 302 and Q' point 304. A PQ-time interval 316 is the time interval between a sensed P-wave 302 and a sensed Q point 306. A PR-time interval 318 is the time interval between a sensed P-wave 302 and a sensed R point 308. A PS-time interval 320 is the time interval between a sensed P-wave 302 and a sensed S point 310. A PS'-time interval 322 is the time interval between a sensed P-wave 302 and S' point 312. In one embodiment, the microprocessor 58 receives the sensed P-wave and QRS complex and determines the time intervals between the wave portions of the cardiac cycle. In an alternative embodiment, surface ECG measurements are made, thought the use of a 2-point lead surface ECG, from which the time intervals between the wave portions of the cardiac cycle are determined.

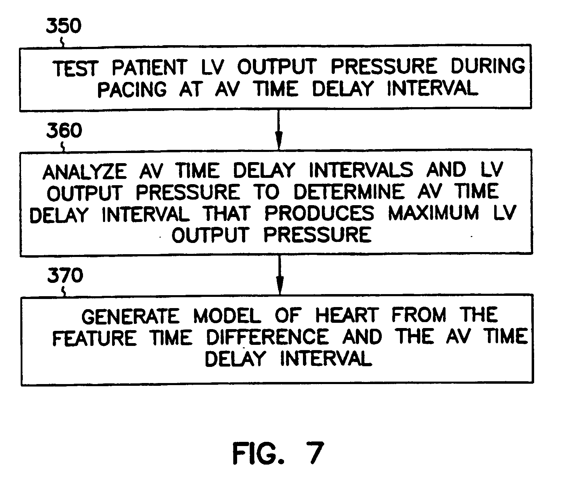

Referring to now to FIG. 7, there is shown a flow chart of one embodiment of the present invention for determining a model of the heart. In one embodiment, the model of the heart is used to determine an estimated optimal AV time delay interval. At step 350, a plurality of patients are tested, where each of the patient's have cardiac pacing pulses delivered to a ventricular chamber of the patient's heart. The cardiac pacing pulses are delivered at a plurality of predetermined AV time delay intervals. In one embodiment, the AV time delay intervals are measured from the occurrence of the patient's P-wave. For each patient, LV+dp/dt values are measured and recorded for each of the plurality of AV time delay intervals tested. In an alternative embodiment, the aortic pulse pressure is measured and recorded for each of the plurality of AV time delay intervals tested. In one embodiment, the data was acquired from patients who were paced at the left ventricular free wall with 5 different AV delay time intervals, where the AV delay time intervals were delivered in a randomized order.

In one embodiment, this type of testing is performed on patients who exhibit a common cardiac condition. For example, in the present embodiment the patients tested were all CHF patients. In addition, the cardiac pacing pulses used in determining the model can be delivered to any number of locations in the ventricular region of the heart. In one embodiment, the cardiac pacing pulses are delivered to an epicardial location on the patient's left ventricular chamber, such as at the left ventricular free wall as previously mentioned. In an alternative embodiment, the cardiac pacing pulses are delivered to an endocardial location that is adjacent to the patient's left ventricular chamber. In an additional embodiment, the cardiac pacing pulses are delivered to an endocardial location in the patient's right ventricular chamber. Additionally, different combinations of right ventricular and left ventricular pacing can be used in determining, or testing for, the maximum left ventricular systolic performance.

In addition to providing cardiac pacing pulses at a number of locations in the ventricular region of the heart, intrinsic electrocardiograms signals are also recorded during the testing of the predetermined AV time delay intervals. In one embodiment, the intrinsic electrocardiogram signals are recorded epicardially from the left ventricular free wall. In one embodiment, the intrinsic electrocardiograms are measured and recorded through the use of a standard 12-point lead surface ECG measurement. The electrocardiograms were digitized (sampling frequency: 500 Hz; resolution 14 bit) and analyzed offline using specially designed software.

At step 360, the times for the AV time delay interval and the corresponding left ventricular systolic performance measurements from each of the patients are analyzed. Each patient's data is analyzed to determine the AV time delay interval that produced the maximum left ventricular systolic performance. For each patient, the predetermined AV time delay interval producing the maximum left ventricular systolic performance is recorded and stored for use in determining the model of the heart. Along with determining the AV time delay that produces the maximum left ventricular systolic performance, time differences between predetermined features on the electrocardiogram signals recorded during the test are also determined. In one embodiment, the electrocardiogram signals recorded during the test are used to determine a feature time difference at step 360.

In one embodiment, the feature time difference is determined between a first predetermined feature on a sensed P-wave and a second predetermined feature on a sensed QRS-complex from the patient's heart that has been paced at the predetermined AV time delay interval that produced a maximum left ventricular systolic performance. In one embodiment, the feature time difference is derived from the atrial sensing or pacing mark of the sensed P-wave and the beginning portion of the sensed QRS-complex (Q* point) from a left ventricular electrocardiogram signal. This value is the PQ*-time interval 314 as previously described. In an alternative embodiment, the feature time difference is derived from an atrial sensing or pacing mark of the sensed P-wave and a maximum deflection point of an R point of the sensed QRS-complex from a left ventricular electrocardiogram signal. This value is the PR-time interval 318 as previously described. In an additional embodiment, the PR-interval feature time difference is derived from a right ventricular electrocardiogram signal. Measured PQ'-time interval 314 and PR-time interval 318 values from normal QRS complexes were averaged and then used to derive the model for determining the estimated optimal AV time delay intervals. In addition, other feature time differences exist which could be used in determining a model of the heart from determining estimated optimal AV time delay interval values.

For the CHF patients, time values for Q' points 304, Q points 306, R points 308, S points 310, and S' points 312 from the QRS complexes were determined and the PQ'-time interval 314, PQ-time interval 316, PR-time interval 318, PS-time interval 320, and PS'-time interval 322 were calculated. An average for each of the PQ'-time interval 314, PQ-time interval 316, PR-time interval 318, PS-time interval 320, and PS'-time interval 322 was then calculated along with their respective standard deviation. Values of a measured time interval that were at least one standard deviation away from the mean were removed and the remaining time interval data points were averaged again to obtain final mean for the PQ'-time interval 314, PQ-time interval 316, PR-time interval 318, PS-time interval 320, and PS'-time interval 322.

At step 370, a model of the heart is generated from the feature time difference and the predetermined AV time delay interval that produced the maximum left ventricular systolic performance. In one embodiment, the model is generated from the relationship of the feature time differences and the predetermined AV time delay intervals for the patients. One way of determining this relationship is to map the patients predetermined AV time delay interval data versus the corresponding feature time difference on a Cartesian coordinate system. Based on the mapped data, a model of the heart is derived that is subsequently used in determining an estimated optimal AV time delay interval. In one embodiment, the model of the heart for determining an estimated optimal AV time delay interval is a linear model. In one embodiment, the model is used in an electrical pulse generating device to provide therapy to the heart, such as providing pacing pulses to CHF patient's to improve their cardiac output efficiency.

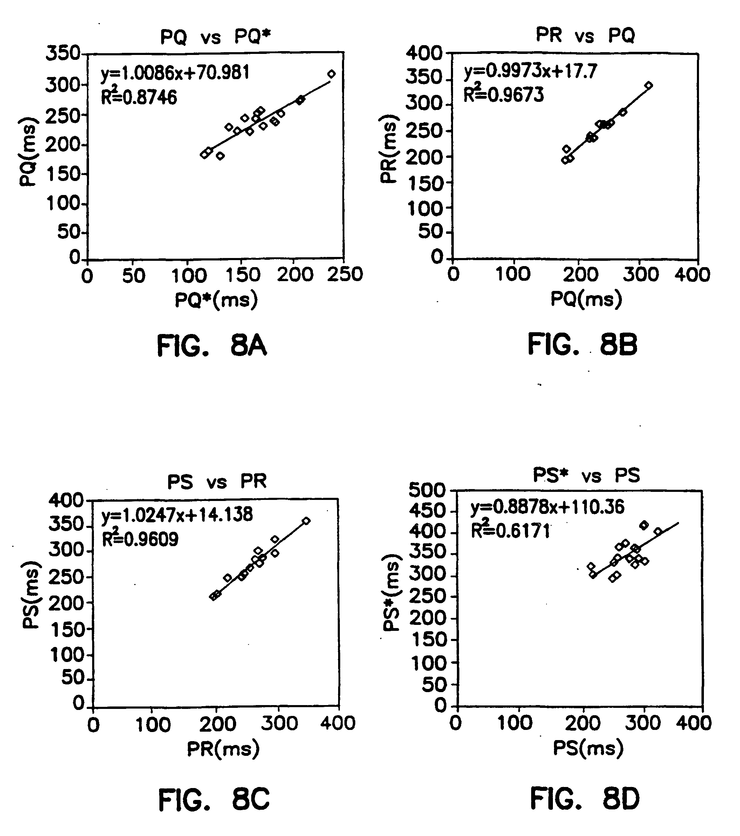

Referring now to FIG.s 8A, 8B, 8C and 8D there is shown plots demonstrating correlations between pairs of the PQ'-time interval 314, PQ-time interval 316, PR-time interval 318, PS-time interval 320, and PS'-time interval 322 for the CHF patients. Data collected from CHF patients indicates there is a high correlation between PQ'-time interval 314 and PQ-time interval 316 (FIG. 8A); PR-time interval 318 and PQ-time interval 316 (FIG. 8B); and PS-time interval 322 and PR-time interval 318 (FIG. 8C). These correlations indicate that the time intervals from the Q' points 304 to the Q points 306; from the Q points 306 to the R points 308; and from the R points 308 to the S points 310 are nearly independent of the PQ'-time interval 314, PQ-time interval 316, PR-time interval 318, PS-time interval 320, and PS'-time interval 322. In other words, the location of P-wave 302 has a limited effect on the inter-difference between the Q' points 304, the Q points 306, the R points 308, and the S points 310.

The data from FIG.s 5F and 8 indicates that the electrical activation pattern of the left ventricular is highly similar for the tested patients. Once the cardiac signal has traveled into the Purkinje's network and into ventricles, which coincide with the beginning of the QRS complex and the stimulation of the ventricles, the remainder of the cardiac cycle was similar for the patients under the study. Based on these observations, specific electrical activation patterns required to achieve optimal LV synchrony have been found to be similar among the patients. The time interval between the beginning of the electrical activation in the LV (as indicated by Q') and the pacing mark may be similar among the patients at their optimal AV time delay interval for LV synchrony. As a result, differences in optimal AV time delay intervals for LV synchrony may be largely due to the differences from the P-wave to the beginning of the electrical activation of the left ventricle (i.e., PQ').

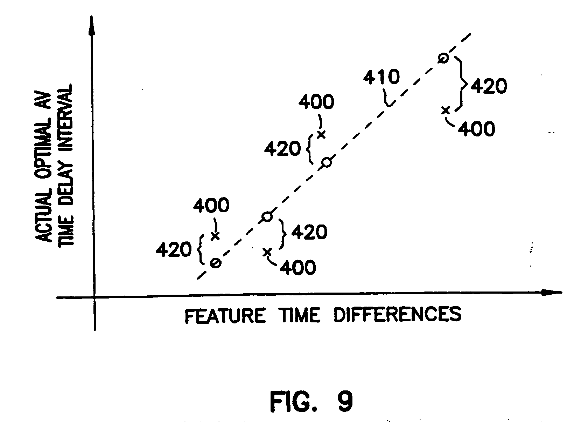

Referring now to FIG. 9 there is shown a Cartesian coordinate system for mapping predetermined AV time delay interval values against feature time differences. Actual data points 400 collected for each of the patients are mapped on the coordinate system. Based on the actual data points 400 mapped on the coordinate system, a model representing the data points 400 is generated. In one embodiment, the model generated is a linear model which is determined from a performing a linear regression on the actual data points 400. The linear model is used to generate a trend line 410 on the coordinate system. In one embodiment, the trend line 410 represents a best overall relationship between the actual optimal AV time delay interval values and the values of the feature time differences. In one embodiment, modeling the data through linear regression allows the mean square differences, 420, between the actual and estimated AV delays to be minimized. The better the correlation between actual data and the model representing the data points, the better the trend line 410 will represent the relationship between the data.

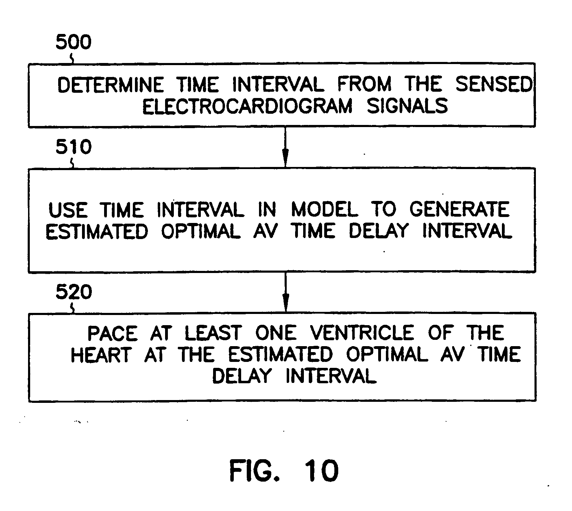

Referring to FIG. 10, there is shown a flow diagram of one embodiment of the present invention. The cardiac signal circuitry housed within the implantable cardiac pacemaker 22 senses the occurrence of the patient's electrocardiogram signals. At step 500, the microprocessor determines a time interval from features on the sensed electrocardiogram signals. In one embodiment, the microprocessor determines the time interval from sensed P-waves and QRS-complexes. At step 510, the microprocessor uses the time interval in a model, such a linear model to generate an estimated optimal AV time delay interval. Then at step 520, the pacing output circuitry of the implantable cardiac pacemaker 22 is used to generate a pacing pulse based on the estimated optimal AV time delay interval to enhance a left ventricular contractility (LV+dP/dt).

Referring now to FIG.s 11A and 11B there is shown mappings of the relationship between the feature time differences in the electrocardiogram signals and the predetermined AV time delay intervals that produced the maximum left ventricular systolic performances (LV dp/dt) for the tested patients. From these mappings, models are generated that express the relationship of the feature timing differences to estimated optimal AV time delay intervals. In one embodiment, the models based on FIG.s 11A and 11B represent the best overall relationship between the actual optimal AV time delay interval and the values of the PQ'-time interval 314 and the PR-time interval 318, respectively.

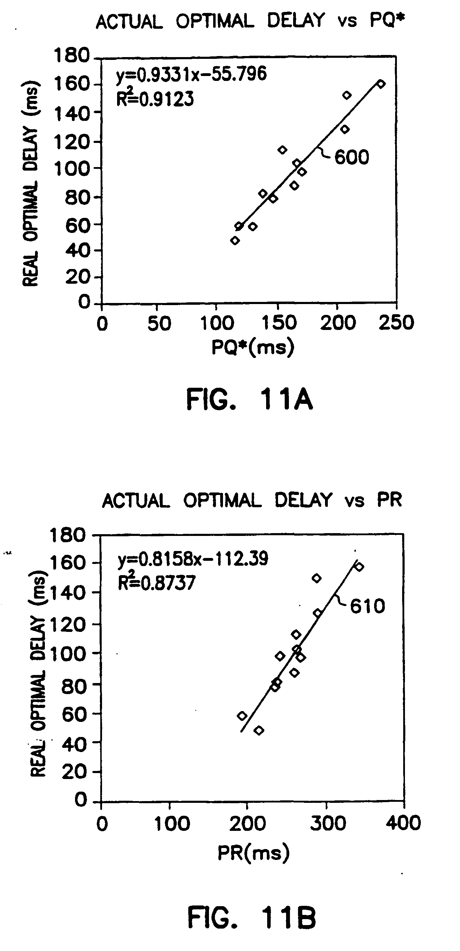

In one embodiment, FIG. 11A shows a mapping of the correlation between the PQ'-time interval 314 and the predetermined AV time delay interval that produced the maximum left ventricular systolic performance. Line 600 on FIG. 11A was derived by linear regression based on the plotted data points. Since the line 600 is linear, the estimated optimal AV time delay interval for LV dp/dt can be written as a linear function of the PQ'-time interval 314 where:

$$AVD_{opt\text{-}\frac{dP}{dt}} = k1 \cdot PQ' - k2 \tag{1}$$

Where AVD is an estimated optimal AV time delay interval, $k1$ is a first coefficient, $k2$ is a second coefficient, and PQ* is a time interval derived from features on an electrocardiogram signal. In one embodiment, PQ* is the PQ*-time interval 314. Additionally, the first coefficient and the second coefficient are empirically derived from the mapping of the time interval (in this instance the PQ*-time interval) and an optimal AV time delay interval.

In one embodiment, equation (1) is utilized within the electronic control circuitry 50 of the implantable cardiac pacemaker 22 to determine an estimated optimal AV time delay interval from the patient's sensed electrocardiogram signals. In one embodiment, equation (1) is used to determine an optimal AV time delay interval based on the patient's electrocardiogram sensed from an atrial and a left ventricular location. In one embodiment, the cardiac signal circuitry housed within the implantable cardiac pacemaker 22 senses the occurrence of both the patient's P-wave and the Q*point during a cardiac cycle. The microprocessor 58 receives the sensed P-wave and Q* point and determines a time interval between these two features. The time interval is then used in equation (1) to generate the AV time delay interval. The implantable cardiac pacemaker 22 then provides pacing pulses to at least one ventricle of the patient's heart at the calculated AV time delay interval to enhance the left ventricular peak ejection pressure. In one embodiment, pacing at the left ventricular location is accomplished through the use of the ventricular catheter, 26 or 152. In an additional embodiment, the pacing pulses generated by the implantable cardiac pacemaker based on equation (1) are generated in real time from the patient's sensed electrocardiogram signals.

In one embodiment, when equation (1) is used to determine the estimated optimal AV time delay interval and the pacing pulses are delivered to a left ventricular location, the first coefficient, $k1$, has a value that is approximately equal to 0.93 and the second coefficient, $k2$, has a value that is approximately equal to 55.8. It is intended that these coefficient values are not limited to the values shown, and any coefficient values derived from the relationship between actual optimal AV time delay intervals and features on electrocardiogram signals are considered to be within the scope of the present invention.

In an additional embodiment, FIG. 11B shows a mapping of the correlation between the PR-time interval 318 and the predetermined AV time delay interval that produced the maximum left ventricular systolic performance. Line 610 on FIG. 11B was derived by linear regression based on the plotted data points. Since the line 610 is linear, the estimated optimal AV time delay interval for LV dp/dt can be written as a linear function of the PR-time interval 318 where:

$$AVD_{opt\text{-}\frac{dP}{dt}} = k1 \cdot PR - k2 \qquad (2)$$

Where AVD is an estimated optimal AV time delay interval, $k1$ is the first coefficient, $k2$ is the second coefficient, and PR is a time interval derived from features on an electrocardiogram signal. In one embodiment, PR is the PR-time interval 318. Additionally, the first coefficient and the second coefficient are empirically derived from the mapping of the time interval (in this instance the PR-time interval) and an optimal AV time delay interval.

In one embodiment, equation (2) is utilized within the electronic control circuitry 50 of the implantable cardiac pacemaker 22 to determine an estimated optimal AV time delay interval from the patient's sensed electrocardiogram signals. In one embodiment, equation (2) is used to determine an optimal AV time delay interval based on the patient's electrocardiogram sensed from an atrial and a left ventricular location. The estimated optimal AV delay interval from equation (2) is then used to time the delivery of a pacing pulse to the patient's left ventricle. In one embodiment, pacing at the left ventricular location is accomplished through the use of the ventricular catheter, 26 or 152. In an additional embodiment, the pacing pulses generated by the implantable cardiac pacemaker based on equation (2) are generated in real time from the patient's sensed electrocardiogram signals.

In one embodiment, when equation (2) is used to determine the estimated optimal AV time delay interval and the pacing pulses are delivered to a left ventricular location, the first coefficient, $k1$, has a value that is approximately equal to 0.82 and the second coefficient, $k2$, has a value that is approximately equal to 112.4. It is intended that these coefficient values are not limited to the values shown, and any coefficient values derived from the relationship between actual optimal AV time delay intervals and features on electrocardiogram signals are considered to be within the scope of the present invention.

Referring now to FIG.s 12A and 12B, there are shown plots of estimated optimal AV time delay intervals as a function of actual optimal AV time delay intervals. The estimated optimal AV time delay interval values were calculated using equation (1) and (2), where the feature time differences were determined from the patient's sensed atrial and left ventricular electrocardiograms. Table 1 shows the differences between the estimated optimal AV time delay interval determined using equation (1) and the actual optimal AV time delay interval using the PQ*-time interval 314 for CHF patients.

Table 1

| Differences Between Estimated and Actual Optimal AV time delay interval | | | |
|---|---|---|---|
| Patient | Actual Optimal AV time delay interval (for dp/dt) (ms) | Estimated Optimal AV time delay interval Using PQ', $k1$ = 0.93, $k2$ = 55.8 | Relative Difference (%) (= 100\| (actual-estimated)/ actual \|) |
| 2 | 78 | 71 | 9 |
| 3 | 45 | 50 | 12 |
| 5 | 150 | 134 | 11 |
| 6 | 95 | 98 | 3 |
| 7 | 158 | 162 | 3 |
| 8 | 75 | 28 | 4 |
| 9 | 110 | 85 | 23 |

Table 1   (continued)

| Differences Between Estimated and Actual Optimal AV time delay interval | | | |
|---|---|---|---|
| Patient | Actual Optimal AV time delay interval (for dp/dt) (ms) | Estimated Optimal AV time delay interval Using PQ', $k1$ = 0.93, $k2$ = 55.8 | Relative Difference (%) (= 100\| (actual-estimated)/ actual \|) |
| 10 | 100 | 96 | 4 |
| 11 | 125 | 133 | 6 |
| 12 | 55 | 63 | 14 |
| 15 | 55 | 53 | 3 |
| 16 | 95 | 100 | 6 |
| 18 | 85 | 95 | 12 |
| Mean = 8%, STD = 6% | | | |

Table 2 shows the differences between the estimated optimal AV time delay interval determined using equation (2) and the actual optimal AV time delay interval using the PR-time interval 318 for CHF patients.

Table 2

| Differences Between Estimated and Actual Optimal AV time delay interval | | | |
|---|---|---|---|
| Patient | Actual Optimal AV time delay interval (for dp/dt) (ms) | Estimated Optimal AV time delay interval Using PR, $k1$ = 0.82, $k2$ = 112.4 | Relative Difference (%) (= 100 \| (actual-estimated)/ actual \|) |
| 2 | 78 | 83 | 6 |
| 3 | 45 | 64 | 42 |
| 5 | 150 | 123 | 18 |
| 6 | 95 | 107 | 13 |
| 7 | 158 | 166 | 5 |
| 8 | 75 | 81 | 8 |
| 9 | 110 | 103 | 6 |
| 10 | 100 | 104 | 4 |
| 11 | 125 | 124 | 0.4 |
| 12 | 55 | 46 | 16 |
| 15 | 55 | 49 | 10 |
| 16 | 95 | 85 | 10 |
| 18 | 85 | 100 | 18 |
| Mean = 12%, STD = 11% | | | |

In one embodiment, the first coefficient and the second coefficient for equation (1) and equation (2) are determined from line 430 and line 440, respectively, and are, therefore, dependent upon the data points on which the line is drawn. The sensitivity of the coefficients was tested with respect to a change in the size of the data group. Different numbers of data points (i.e., number of patients) were used to generate line 430 in a plot of actual optimal AV time delay interval versus the PQ'-time interval 314, and to generate line 440 in a plot of actual optimal AV time delay interval versus the PR-time interval 318. Values for the first coefficient, $k1$, and the second coefficient, $k2$, were obtained from the line 430 equation in the plot for PQ' and from the line 440 equation in the plot for PR.

| Equation (1) | | | | Equation (2) | | | |
|---|---|---|---|---|---|---|---|
| No. of data points | $k1$ | $k2$ | $r^2$ | No. of data points | $k1$ | $k2$ | $r^2$ |
| 2 | 1.47 | 123.1 | 1 | 2 | 1.44 | 265.8 | 1 |
| 3 | 1.14 | 82.2 | 0.99 | 3 | 1.46 | 268.8 | 1 |
| 4 | 1.12 | 81.0 | 0.97 | 4 | 1.32 | 240.1 | 0.92 |
| 5 | 0.965 | 59.7 | 0.90 | 5 | 0.946 | 149.7 | 0.88 |
| 6 | 0.974 | 61.9 | 0.96 | 6 | 0.942 | 148.4 | 0.89 |
| 7 | 0.943 | 53.3 | 0.91 | 7 | 0.941 | 146.6 | 0.88 |
| 8 | 0.943 | 53.4 | 0.92 | 8 | 0.941 | 146.8 | 0.88 |
| 9 | 0.904 | 48.3 | 0.91 | 9 | 0.941 | 146.9 | 0.88 |
| 10 | 0.938 | 55.1 | 0.91 | 10 | 0.854 | 122.3 | 0.88 |
| 11 | 0.937 | 55.0 | 0.92 | 11 | 0.828 | 114.9 | 0.89 |
| 12 | 0.934 | 55.1 | 0.92 | 12 | 0.822 | 112.4 | 0.89 |
| 13 | 0.933 | 55.8 | 0.91 | 13 | 0.817 | 112.4 | 0.87 |
| 14 | 0.943 | 57.7 | 0.92 | 14 | 0.841 | 120.3 | 0.85 |

After five data points, the $k1$ and $k2$ values for equation (1) did not change significantly. The value of $k1$ oscillates between 0.904 and 0.974, and $k2$ oscillates between 48.3 and 61.9. On the other hand, the $k1$ and $k2$ values for equation (2) experienced a continuous delay as the number of data points increased. As a result, the $k1$ and $k2$ values used in equation (1) are less sensitive to the number of data points than the $k1$ and $k2$ values used in equation (2). So, in one embodiment, the PQ'-time interval 314, as used in equation (1), is more accurate and robust to predict an optimal AV time delay interval than using the PR-time interval 318 in equation (2). In addition, observing the mean difference between the actual and estimated optimal AV time delay interval of Tables 1 and 2, using the PQ'-time interval 314 provides a more accurate result that using the PR-time interval 318. However, the PR-time interval 318 is more easily measured using an implantable medical system 20, such as the implantable cardiac pacemaker 22 previously described or an implantable defibrillator, than the PQ'-time interval 314.

In an additional embodiment, equation (2) is also used to determine estimated optimal AV time delay intervals when electrocardiogram signals are sensed from an atrial and at least a right ventricle location. Based on a mapping of the relationship between the PR-time interval feature time difference in the right ventricular electrocardiogram signals and the predetermined AV time delay intervals that produced the maximum left ventricular systolic performances (LV dp/dt) for the tested patients, a linear model having the form of equation (2) was generated. The line representing the linear model was derived by linear regression based on the plotted data points.

In one embodiment, equation (2) is utilized within the electronic control circuitry 50 of the implantable cardiac pacemaker 22 to determine an estimated optimal AV time delay interval from the patient's sensed electrocardiogram signals. In one embodiment, equation (2) is used to determine an optimal AV time delay interval based on the patient's electrocardiogram sensed from an atrial and at least a right ventricular location. The estimated optimal AV delay interval from equation (2) is then used to time the delivery of a pacing pulse to the patient's right ventricle. In one embodiment, pacing at the right ventricular location is accomplished through the use of the ventricular catheter, 100 or 150. In an additional embodiment, the pacing pulses generated by the implantable cardiac pacemaker based on equation (2) are generated in real time from the patient's sensed electrocardiogram signals.

In an alternative embodiment, the optimal AV delay interval determined using equation (2) is used to time the pacing of the heart from a left ventricular location. In an additional embodiment, the optimal AV delay interval determined using equation (2) is used to time the pacing of the heart from both a right ventricular and a left ventricular location. In one embodiment, the medical device system 20, as previously described, is used to provide pacing pulses to at least one of a right ventricle and a left ventricle of the heart.

In one embodiment, when equation (2) is used to determine the estimated optimal AV time delay interval and the pacing pulses are delivered to at least a right ventricular and a left ventricular location, the first coefficient, $k1$, has a value that is approximately equal to 0.5 and the second coefficient, $k2$, has a value that is approximately equal to 30.0. It is intended that these coefficient values are not limited to the values shown, and any coefficient values derived from the relationship between actual optimal AV ume delay intervals and features on electrocardiogram signals are considered to be within the scope of the present invention.

**Conclusion**

The present pacing system may be employed in a variety of pacing devices, including implantable pacing devices. The present system may be used for pacing one or more ventricles. A variety of pacing electrode configurations may be employed without departing from the present invention including multiple pacing sites at a ventricle(s), provided that the required electrical or mechanical events are monitored. Changes in the coefficients and order of methods provided herein may be practiced accordingly without departing from the scope of the present invention.

**Claims**

1.  An apparatus (20) comprising:

    a programmable pulse generator (22) operable to transmit ventricular pacing pulses with an atrio-ventricular delay calculated from a measured time interval (314,316,318,320,322), said measured time interval being measured during a nonpaced systolic cycle by the programmable pulse generator between a first event (302) related to an atrial contraction and a second event (304,306,308,310,312) related to a ventricular contraction; **characterized in that** the calculated atrio-ventricular delay provided by said programmable pulse generator is determined as an approximation for an optimal atrio-ventricular delay using a predetermined mapping of a relationship of measured time intervals to respective optimal atrio-ventricular delays based on sampled data from a plurality of patients, the sampled data including a respective optimal atrio-ventricular delay to optimise ventricular performance and respective measured time interval for each of the plurality of patients.

2.  The apparatus according to claim 1, wherein the optimal atrio-ventricular delay is an optimal atrio-ventricular delay $AVD_c$ for maximum peak positive left ventricle pressure change during systole.

3.  The apparatus according to claim 2, wherein the first electrical event is a P-wave, the second electrical event is a beginning (Q*) of a QRS complex, and the measured time interval is an interval PQ* between the P-wave and the beginning of the QRS complex.

4.  The apparatus according to claim 3, wherein $AVD_c$ is calculated from a linear equation:

$$AVD_c = K1\ (PQ^*) - K2.$$

5.  The apparatus according to claim 4, wherein K1 is approximately 0.94 and K2 is approximately 55.7 milliseconds.

6.  The apparatus according to claim 4, wherein K1 is approximately 1.0 and K2 is approximately 67 milliseconds.

7.  The apparatus according to claim 4, wherein the interval PQ* is measured using the programmable pulse generator having an electrode in a right atrium and an electrode sensing signals from a left ventricle.

8.  The apparatus according to claim 7, wherein the P-wave is detected when it reaches 20 percent of a maximum P-wave amplitude in the right atrium.

9.  The apparatus according to claim 7, comprising a low pass filter for detecting the beginning of the QRS complex.

10. The apparatus according to claim 9, wherein the beginning of the QRS complex is detected when a slope of a Q-wave reaches 2 percent of its maximum absolute value of the Q-wave slope.

11. The apparatus according to claim 4, wherein the interval PQ* is measured using surface electrocardiogram.

12. The apparatus according to claim 2, wherein the first electrical event is a P-wave, the second electrical event is a peak of an R-wave, and the measured time interval is an interval PR between the P-wave and the peak of the R-wave.

**13.** The apparatus according to claim 12, wherein $AVD_c$ is calculated from a linear equation:

$$AVD_c = N1\ (PR) - N2.$$

**14.** The apparatus according to claim 13, wherein N1 is approximately 0.82 and N2 is approximately 112 milliseconds.

**15.** The apparatus according to claim 13, wherein N1 is approximately 1.0 and N2 is approximately 159 milliseconds.

**16.** The apparatus according to claim 13, wherein PR is measured using the programmable pulse generator having an electrode in a right atrium and an electrode sensing signals from a left ventricle.

**17.** The apparatus according to claim 16, wherein a P-wave is detected when it reaches 20 percent of a maximum P-wave amplitude in the right atrium.

**18.** The apparatus according to claim 2, wherein the first electrical event is a P-wave, the second electrical event is an electrical signal Y signalling an onset of ventricular pressure during systole and the measured time interval is an interval PY between the P-wave and the electrical signal Y.

**19.** The apparatus according to claim 18, wherein $AVD_c$ is calculated from a linear equation:

$$AVD_c = M1\ (PY) - M2.$$

**20.** The apparatus according to claim 19, wherein M1 is approximately 0.96 and M2 is approximately 139 milliseconds.

**21.** The apparatus according to claim 19, wherein M1 is approximately 1.0 and M2 is approximately 150 milliseconds.

**22.** The apparatus according to claim 18, wherein PY is measured using the programmable pulse generator having an electrode in a right atrium and the micromanometer in a left ventricle.

**23.** The apparatus according to claim 22, wherein a P-wave is detected when it reaches 20 percent of a maximum P-wave amplitude in the right atrium.

**24.** The apparatus according to claim 18, wherein the electrical signal Y is provided by a cardiac phonogram.

**25.** The apparatus according to claim 18, wherein the electrical signal Y is provided by an accelerometer.

**26.** The apparatus according to claim 18, wherein the electrical signal Y is provided by a Doppler recording.

**27.** The apparatus according to claim 18, wherein the electrical signal Y is provided by an echo imager.

**28.** The apparatus according to claim 1, wherein the desired atrio-ventricular delay comprises an atrio-ventricular delay $AVD_{cs}$ which provides an approximately optimal atrio-ventricular delay for both contractility and stroke volume calculated by the programmable pulse generator from a predetermined mapping of a relationship of the measured time interval to a near optimal atrio-ventricular delay for maximum peak positive left ventricle pressure change during systole, and a relationship of pulse pressure to normalized atrio-ventricular delays.

**29.** The apparatus according to claim 28, wherein the first electrical event is a P-wave, the second electrical event is a right ventricular sensing marker $R_m$, and the measured time interval is an interval $PR_m$ between the P-wave and the marker $R_m$.

**30.** The apparatus according to claim 29, wherein the atrio-ventricular delay $AVD_{cs}$ is calculated from a linear equation:

$$AVD_{cs} = K3\ PR_m - K4\ \text{milliseconds.}$$

**31.** The apparatus according to claim 30, wherein K3 is 0.5 and K4 is 15 milliseconds.

32. The apparatus according to claim 30, wherein K3 is in a range from 0.4 to 0.6 and K4 is in a range from 0 to 30 milliseconds.

33. The apparatus according to claim 1, wherein the optimal atrio-ventricular delay includes an atrio-ventricular delay $AVD_s$ for maximum pulse pressure calculated by the programmable pulse generator from a predetermined mapping of a relationship of the measured time interval to an optimal atrio-ventricular delay for maximum peak positive left ventricle pressure change during systole to provide an approximation of the atrio-ventricular delay for pacing the ventricle to provide optimal stroke volume.

34. The apparatus according to claim 33, wherein the first event P is a P-wave, the second electrical event is a pulse pressure event x, and the measured time interval is an interval PX is between the P-wave and the pulse pressure event X.

35. The apparatus according to claim 34, wherein the atrio-ventricular delay $AVD_s$ is calculated from a linear equation:

$$AVD_s = K3\ PX - K4\ \text{milliseconds.}$$

36. The apparatus according to claim 35, wherein K3 is 1.22 and K4 is 132 milliseconds.

37. An apparatus according to claim 1, wherein the programmable pulse generator includes an electrical event sensor operable to monitor a signal representing ventricular electrical activity, wherein the programmable pulse generator operable to determine a ventricular electrical event Z which has an approximately constant timing relationship to delivery of ventricular pacing that maximizes peak positive ventricular pressure change, and to deliver a pacing pulse using a timing of the event Z.

38. The apparatus of claim 37, wherein the signal representing ventricular electrical activity comprises an intracardiac electrogram recorded endocardially or epicardially from a ventricle.

39. The apparatus of claim 37, wherein the signal representing ventricular electrical activity comprises 12-lead surface ECG.

40. The apparatus of claim 37, wherein the event is a beginning Q* of a QRS complex.

41. The apparatus of claim 37, wherein the event is a marker signal delivered by an external or implantable device.

42. The apparatus according to claim 1, wherein the programmable pulse generator has an electrical event sensor that monitors a signal representing atrial mechanical activity, wherein the programmable pulse generator determines an atrial mechanical event, X, which has a constant timing relationship to the peak of atrial systole, and delivers a pacing pulse using a timing of event X.

43. The apparatus according to claim 1, wherein the programmable pulse generator is adapted to switch among:

transmitting ventricular pacing pulses to maximize contractility using an atrio-ventricular delay $AVD_c$ for maximum peak positive LV pressure change during systole;
transmitting ventricular pacing pulses to maximize stroke volume using an atrio-ventricular delay $AVD_s$ for maximum pulse pressure; and
transmitting ventricular pacing pulses to nearly optimize contractility and stroke volume using an atrio-ventricular delay $AVD_{cs}$ for balancing peak positive LV pressure change during systole.

44. The apparatus according to claim 43, wherein the atrio-ventricular delay $AVD_c$ is calculated from a linear equation

$$AVD_c = K1\ (PQ^*) - K2,$$

wherein PQ* represents a measured time interval between a P-wave and a beginning of a QRS complex, and wherein constants K1 and K2 are derived from sampled data from a plurality of patients.

**45.** The apparatus according to claim 43, wherein the atrio-ventricular delay $AVD_c$ is calculated from a linear equation

$$AVD_c = N1 \, (PR) - N2,$$

wherein PR represents a measured time interval between a P-wave and an R-wave, and wherein constants N1 and N2 are derived from sampled data from a plurality of patients.

**46.** The apparatus according to claim 43, wherein the atrio-ventricular delay $AVD_c$ is calculated from a linear equation

$$AVD_c = M1 \, (PY) - M2,$$

wherein PY represents a measured time interval between a P-wave and an onset of ventricular pressure during systole, and wherein constants M1 and M2 are derived from sampled data from a plurality of patients.

**47.** The apparatus according to any of claims 43 to 46, wherein the atrio-ventricular delay $AVD_s$ is calculated from a linear equation

$$AVD_s = K3 \, (PX) - K4,$$

wherein PX represents a measured time interval between a P-wave and a pulse pressure, and wherein constants K3 and K4 are derived from sampled data from a plurality of patients.

**48.** The apparatus according to any of claims 43 to 47, wherein the atrio-ventricular delay $AVD_{cs}$ is calculated from a linear equation

$$AVD_{cs} = K3 \, (PR_m) - K4,$$

wherein $PR_m$ represents a measured time interval between a P-wave and a right ventricular sensing marker, and wherein constants K3 and K4 are derived from sampled data from a plurality of patients.

**49.** The apparatus according to any of claims 43 to 48, wherein the programmable pulse generator is adapted to default to the atrio-ventricular delay $AVD_{cs}$, to switch to the atrio-ventricular delay $AVD_c$ to improve contractility and to switch to atrio-ventricular delay $AVD_s$ to improve stroke volume.

**Patentansprüche**

**1.** Gerät (20) mit
einem programmierbaren Pulsgenerator (22), der zum Übertragen ventrikulärer Stimulationspulse mit einer atrioventrikulären Verzögerung, die aus einem gemessenen Zeitintervall (316, 318, 320, 322) berechnet ist, betreibbar ist, wobei das gemessene Zeitintervall während eines nicht-stimulierten systolischen Zyklus durch den programmierbaren Pulsgenerator zwischen einem ersten Ereignis (302) in Bezug auf eine Vorhofkontraktion und einem zweiten Ereignis (304, 306, 308, 310, 312) in Bezug auf eine Ventrikelkontraktion gemessen wird, **dadurch gekennzeichnet, daß** die von dem programmierbaren Pulsgenerator bereitgestellte, berechnete atrioventrikuläre Verzögerung als eine Näherung für eine optimale atrioventrikuläre Verzögerung unter Verwendung einer vorbestimmten Zuordnung einer Beziehung gemessener Zeitintervalle zu zugehörigen optimalen atrioventrikulären Verzögerungen auf Grundlage von Testdaten von mehreren Patienten bestimmt wird, wobei die Testdaten eine zugehörige optimale atrioventrikuläre Verzögerung zum Optimieren der ventrikulären Leistung und ein zugehöriges gemessenes Zeitintervall für jeden der mehreren Patienten aufweisen.

**2.** Gerät nach Anspruch 1, wobei die optimale atrioventrikuläre Verzögerung in einer optimalen atrioventrikulären Verzögerung $AVD_c$ für einen maximalen Spitzenwert der positiven Druckänderung der linken Herzkammer während der Systole besteht.

**3.** Gerät nach Anspruch 2, wobei das erste elektrische Ereignis eine P-Welle, das zweite elektrische Ereignis der Beginn (Q*) eines QRS-Komplexes und das gemessene Zeitintervall ein Intervall PQ* zwischen der P-Welle und dem Beginn des QRS-Komplexes ist.

**4.** Gerät nach Anspruch 3, wobei $AVD_c$ aus einer linearen Gleichung

$$AVD_c = K1\,(PQ^*) - K2$$

berechnet wird.

**5.** Gerät nach Anspruch 4, wobei K1 etwa 0,94 und K2 etwa 55,7 Millisekunden beträgt.

**6.** Gerät nach Anspruch 4, wobei K1 etwa 1,0 und K2 etwa 67 Millisekunden beträgt.

**7.** Gerät nach Anspruch 4, wobei das Intervall PQ* unter Verwendung des programmierbaren Pulsgenerators mit einer Elektrode in einem rechten Vorhof und einer Elektrode zum Messen von Signalen in einer linken Herzkammer gemessen wird.

**8.** Gerät nach Anspruch 7, wobei die P-Welle erfaßt wird, wenn sie 20 Prozent einer maximalen P-Wellen-Amplitude in dem rechten Vorhof erreicht.

**9.** Gerät nach Anspruch 7, mit einem Tiefpaßfilter zum Erfassen des Beginns des QRS-Komplexes.

**10.** Gerät nach Anspruch 9, wobei der Beginn des QRS-Komplexes erfaßt wird, wenn eine Steigung einer Q-Welle 2 Prozent des maximalen Absolutwerts der Q-Wellen-Steigung erreicht.

**11.** Gerät nach Anspruch 4, wobei das Intervall PQ* unter Verwendung eines Oberflächenelektrokardiogramms gemessen wird.

**12.** Gerät nach Anspruch 2, wobei das erste elektrische Ereignis eine P-Welle, das zweite elektrische Ereignis ein Spitzenwert einer R-Welle und das gemessene Zeitintervall ein Intervall PR zwischen der P-Welle und dem Spitzenwert der R-Welle ist.

**13.** Gerät nach Anspruch 12, wobei $AVD_c$ aus einer linearen Gleichung

$$AVD_c = N1\,(PR) - N2$$

berechnet wird.

**14.** Gerät nach Anspruch 13, wobei N1 etwa 0,82 und N2 etwa 112 Millisekunden beträgt.

**15.** Gerät nach Anspruch 13, wobei N1 etwa 1,0 und N2 etwa 159 Millisekunden beträgt.

**16.** Gerät nach Anspruch 13, wobei PR unter Verwendung des programmierbaren Pulsgenerators mit einer Elektrode in einem rechten Vorhof und einer Elektrode zum Messen von Signalen in einer linken Herzkammer gemessen wird.

**17.** Gerät nach Anspruch 16, wobei eine P-Welle erfaßt wird, wenn sie 20 Prozent einer maximalen P-Wellen-Amplitude in dem rechten Vorhof erreicht.

**18.** Gerät nach Anspruch 2, wobei das erste elektrische Ereignis eine P-Welle, das zweite elektrische Ereignis ein elektrisches Signal Y, das das Einsetzen des Ventrikeldrucks während der Systole signalisiert, und das gemessene Zeitintervall ein Intervall PY zwischen der P-Welle und dem elektrischen Signal Y ist.

**19.** Gerät nach Anspruch 18, wobei $AVD_c$ aus einer linearen Gleichung

$$AVD_c = M1 \, (PY) - M2$$

berechnet wird.

**20.** Gerät nach Anspruch 19, wobei M1 etwa 0,96 und M2 etwa 139 Millisekunden beträgt.

**21.** Gerät nach Anspruch 19, wobei M1 etwa 1,0 und M2 etwa 150 Millisekunden beträgt.

**22.** Gerät nach Anspruch 18, wobei PY unter Verwendung des programmierbaren Pulsgenerators mit einer Elektrode in einem rechten Vorhof und einem Mikrodruckmesser in einer linken Herzkammer gemessen wird.

**23.** Gerät nach Anspruch 22, wobei eine P-Welle erfaßt wird, wenn sie 20 Prozent einer maximalen P-Wellen-Amplitude in dem rechten Vorhof erreicht.

**24.** Gerät nach Anspruch 18, wobei das elektrische Signal Y von einem Kardiophonogramm bereitgestellt wird.

**25.** Gerät nach Anspruch 18, wobei das elektrische Signal Y von einem Beschleunigungsmesser bereitgestellt wird.

**26.** Gerät nach Anspruch 18, wobei das elektrische Signal Y durch eine Doppleraufzeichnung bereitgestellt wird.

**27.** Gerät nach Anspruch 18, wobei das elektrische Signal Y von einer Echoabbildungseinrichtung bereitgestellt wird.

**28.** Gerät nach Anspruch 1, wobei die gewünschte atrioventrikuläre Verzögerung eine atrioventrikuläre Verzögerung $AVD_{cs}$ aufweist, die eine annähernd optimale atrioventrikuläre Verzögerung sowohl hinsichtlich der Kontraktionsfähigkeit als auch des Schlagvolumens, berechnet durch den programmierbaren Pulsgenerator aus einer vorbestimmten Zuordnung einer Beziehung des gemessenen Zeitintervalls zu einer nahezu optimalen atrioventrikulären Verzögerung für einen maximalen Spitzenwert der positiven Druckänderung in der linken Herzkammer während der Systole und einer Beziehung des Pulsdrucks zu normalisierten atrioventrikulären Verzögerungen, bereitstellt.

**29.** Gerät nach Anspruch 28, wobei das erste elektrische Ereignis eine P-Welle, das zweite elektrische Ereignis eine rechtsventrikuläre Meßmarkierung $R_m$ und das gemessene Zeitintervall ein Intervall $PR_m$ zwischen der P-Welle und der Markierung $R_m$ ist.

**30.** Gerät nach Anspruch 29, wobei die atrioventrikuläre Verzögerung $AVD_{cs}$ aus einer linearen Gleichung

$$AVD_{cs} = K3 \, PR_m - K4 \; \text{Millisekunden}$$

berechnet wird.

**31.** Gerät nach Anspruch 30, wobei K3 0,5 und K4 15 Millisekunden beträgt.

**32.** Gerät nach Anspruch 30, wobei K3 in einem Bereich von 0,4 bis 0,6 und K4 in einem Bereich von 0 bis 30 Millisekunden liegt.

**33.** Gerät nach Anspruch 1, wobei die optimale atrioventrikuläre Verzögerung eine atrioventrikuläre Verzögerung $AVD_s$ für einen maximalen Pulsdruck aufweist, der durch den programmierbaren Pulsgenerator aus einer vorbestimmten Zuordnung einer Beziehung des gemessenen Zeitintervalls zu einer optimalen atrioventrikulären Verzögerung für einen maximalen Spitzenwert der positiven Druckänderung der linken Herzkammer während der Systole berechnet wird, um eine Näherung der atrioventrikulären Verzögerung für eine Herzkammerstimulation hinsichtlich eines optimalen Schlagvolumens bereitzustellen.

**34.** Gerät nach Anspruch 33, wobei das erste Ereignis P eine P-Welle, das zweite elektrische Ereignis ein Pulsdruckereignis X, und das gemessene Zeitintervall ein Intervall PX zwischen der P-Welle und dem Pulsdruckereignis X ist.

**35.** Gerät nach Anspruch 34, wobei die atrioventrikuläre Verzögerung $AVD_s$ aus einer linearen Gleichung

$$AVD_s = K3 \ PX - K4 \ \text{Millisekunden}$$

berechnet wird.

**36.** Gerät nach Anspruch 35, wobei K3 1,22 und K4 132 Millisekunden beträgt.

**37.** Gerät nach Anspruch 1, wobei der programmierbare Pulsgenerator einen elektrischen Ereignissensor aufweist, der zum Überwachen eines Signals betreibbar ist, das die ventrikuläre elektrische Aktivität darstellt, wobei der programmierbare Pulsgenerator dazu betreibbar ist, ein ventrikuläres elektrisches Ereignis Z zu bestimmen, das ein annähernd konstantes Zeitverhältnis zur Bereitstellung der Herzkammerstimulation aufweist, die den Spitzenwert der positiven Druckänderung der Herzkammer maximiert, und einen Stimulationspuls bei zeitlicher Koordinierung des Ereignisses Z zu liefern.

**38.** Gerät nach Anspruch 37, wobei das die ventrikuläre elektrische Aktivität darstellende Signal ein intrakardiales Elektrogramm aufweist, das endokardial oder epikardial an einer Herzkammer aufgezeichnet wurde.

**39.** Gerät nach Anspruch 37, wobei das die ventrikuläre elektrische Aktivität darstellende Signal ein 12-Leitungs-Oberflächen-EKG aufweist.

**40.** Gerät nach Anspruch 37, wobei das Ereignis einen Beginn Q* eines QRS-Komplexes darstellt.

**41.** Gerät nach Anspruch 37, wobei das Ereignis ein Markierungssignal darstellt, das durch eine externe oder implantierbare Vorrichtung geliefert wird.

**42.** Gerät nach Anspruch 1, wobei der programmierbare Pulsgenerator einen elektrischen Ereignissensor aufweist, der ein Signal überwacht, das die mechanische Vorhofaktivität darstellt, wobei der programmierbare Pulsgenerator ein mechanisches Vorhofereignis X bestimmt, das eine konstante Zeitbeziehung zum Spitzenwert der Vorhofsystole aufweist, und einen Stimulationspuls bei zeitlicher Koordinierung des Ereignisses X liefert.

**43.** Gerät nach Anspruch 1, wobei der programmierbare Pulsgenerator dazu ausgelegt ist, umzuschalten zwischen
dem Übertragen ventrikulärer Stimulationspulse zum Maximieren der Kontraktionsfähigkeit unter Verwendung einer atrioventrikulären Verzögerung $AVD_c$ für einen maximalen Spitzenwert der positiven LV-Druckänderung während der Systole,
dem Übertragen ventrikulärer Stimulationspulse zum Maximieren des Schlagvolumens unter Verwendung einer atrioventrikulären Verzögerung $AVD_s$ für einen maximalen Pulsdruck und
dem Übertragen ventrikulärer Stimulationspulse zur näherungsweisen Optimierung der Kontraktionsfähigkeit und des Schrittvolumens unter Verwendung einer atrioventrikulären Verzögerung $AVD_{cs}$ zum Abgleichen eines positiven Spitzenwerts der LV-Druckänderung während der Systole.

**44.** Gerät nach Anspruch 43, wobei die atrioventrikuläre Verzögerung $AVD_c$ aus einer linearen Gleichung

$$AVD_c = K1 \ (PQ^*) - K2$$

berechnet wird, wobei PQ* ein gemessenes Zeitintervall zwischen einer P-Welle und einem Beginn eines QRS-Komplexes darstellt, und die Konstanten K1 und K2 aus Testdaten von mehreren Patienten hergeleitet sind.

**45.** Gerät nach Anspruch 43, wobei die atrioventrikuläre Verzögerung $AVD_c$ aus einer linearen Gleichung

$$AVD_c = N1 \ (PR) - N2$$

berechnet wird, wobei PR ein gemessenes Zeitintervall zwischen einer P-Welle und einer R-Welle darstellt, und die Konstanten N1 und N2 aus Testdaten von mehreren Patienten hergeleitet sind.

**46.** Gerät nach Anspruch 43, wobei die atrioventrikuläre Verzögerung $AVD_c$ aus einer linearen Gleichung

$$AVD_c = M1\,(PY) - M2$$

berechnet wird, wobei PY ein gemessenes Zeitintervall zwischen einer P-Welle und einem Einsetzen des Ventrikeldrucks während der Systole darstellt, und die Konstanten M1 und M2 aus Testdaten von mehreren Patienten hergeleitet sind.

47. Gerät nach einem der Ansprüche 43 bis 46, wobei die atrioventrikuläre Verzögerung $AVD_s$ aus einer linearen Gleichung

$$AVD_s = K3\,(PX) - K4$$

berechnet wird, wobei PX ein gemessenes Zeitintervall zwischen einer P-Welle und einem Pulsdruck darstellt, und die Konstanten K3 und K4 aus Testdaten von mehreren Patienten hergeleitet sind.

48. Gerät nach einem der Ansprüche 43 bis 47, wobei die atrioventrikuläre Verzögerung $AVD_{cs}$ aus einer linearen Gleichung

$$AVD_{cs} = K3\,(PR_m) - K4$$

berechnet wird, wobei $PR_m$ ein gemessenes Zeitintervall zwischen einer P-Welle und einer rechtsventrikulären Meßmarkierung darstellt, und die Konstanten K3 und K4 aus Testdaten von mehreren Patienten hergeleitet sind.

49. Gerät nach einem der Ansprüche 43 bis 48, wobei der programmierbare Pulsgenerator dazu ausgelegt ist, standardmäßig die atrioventrikuläre Verzögerung $AVD_{cs}$ anzunehmen, auf die atrioventrikuläre Verzögerung $AVD_c$ umzuschalten, um die Kontraktionsfähigkeit zu verbessern, und auf die atrioventrikuläre Verzögerung $AVD_s$ umzuschalten, um das Schlagvolumen zu verbessern.

## Revendications

1. Appareil (20) comportant :

   un générateur programmable (22) d'impulsions pouvant être mis en oeuvre pour émettre des impulsions de stimulation ventriculaire avec un retard auriculo-ventriculaire calculé à partir d'un intervalle de temps mesuré (314, 316, 318, 320, 322), ledit intervalle de temps mesuré étant mesuré par le générateur programmable d'impulsions pendant un cycle systolique non stimulé entre un premier événement (302) associé à une contraction auriculaire et un second un événement (304, 306, 308, 310, 312) associé à une contraction ventriculaire ; **caractérisé en ce que** le retard auriculo-ventriculaire calculé établi par ledit générateur programmable d'impulsions est déterminé sous la forme d'une approximation pour un retard auriculo-ventriculaire optimal en utilisant une application prédéterminée d'une relation d'intervalles de temps mesurés sur des retards auriculo-ventriculaires optimaux respectifs basés sur des données échantillonnées provenant de plusieurs patients, les données échantillonnées comprenant un retard auriculo-ventriculaire optimal respectif pour optimiser la performance ventriculaire et un intervalle de temps mesuré respectif pour chacun des multiples patients.

2. Appareil selon la revendication 1, dans lequel le retard auriculo-ventriculaire optimal est un retard auriculo-ventriculaire optimal $AVD_c$ pour une variation de pression du ventricule gauche positive de pic maximal pendant une systole.

3. Appareil selon la revendication 2, dans lequel le premier événement électrique est une onde P, le second événement électrique est un début (Q*) d'un complexe QRS, et l'intervalle de temps mesuré est un intervalle PQ* entre l'onde P et le début du complexe QRS.

4. Appareil selon la revendication 3, dans lequel $AVD_c$ est calculé d'après une équation linéaire :

$$AVD_c = K1 \, (PQ^*) - K2.$$

**5.** Appareil selon la revendication 4, dans lequel K1 est d'environ 0,94 et K2 est d'environ 55,7 millisecondes.

**6.** Appareil selon la revendication 4, dans lequel K1 est d'environ 1,0 et K2 est d'environ 67 millisecondes.

**7.** Appareil selon la revendication 4, dans lequel l'intervalle PQ* est mesuré en utilisant le générateur programmable d'impulsions ayant une électrode dans l'oreillette droite et une électrode captant des signaux du ventricule gauche.

**8.** Appareil selon la revendication 7, dans lequel l'onde P est détectée lorsqu'elle atteint 20 % d'une amplitude d'onde P maximale dans l'oreillette droite.

**9.** Appareil selon la revendication 7, comportant un filtre passe-bas pour détecter le début du complexe QRS.

**10.** Appareil selon la revendication 9, dans lequel le début du complexe QRS est détecté lorsque la pente d'une onde Q atteint 2 % de sa valeur absolue maximale de pente d'onde Q.

**11.** Appareil selon la revendication 4, dans lequel l'intervalle PQ* est mesuré en utilisant un électrocardiogramme de surface.

**12.** Appareil selon la revendication 2, dans lequel le premier événement électrique est une onde P, le second événement électrique est un pic d'une onde R et l'intervalle de temps mesuré est un intervalle PR entre l'onde P et le pic de l'onde R.

**13.** Appareil selon la revendication 12, dans lequel $AVD_c$ est calculé d'après une équation linéaire :

$$AVD_c = N1 \, (PR) - N2.$$

**14.** Appareil selon la revendication 13, dans lequel N1 est d'environ 0,82 et N2 est d'environ 112 millisecondes.

**15.** Appareil selon la revendication 13, dans lequel N1 est d'environ 1,0 et N2 est d'environ 159 millisecondes.

**16.** Appareil selon la revendication 13, dans lequel PR est mesuré en utilisant le générateur programmable d'impulsions ayant une électrode dans l'oreillette droite et une électrode captant des signaux du ventricule gauche.

**17.** Appareil selon la revendication 16, dans lequel une onde P est détectée lorsqu'elle atteint 20 % d'une amplitude d'onde P maximale dans l'oreillette droite.

**18.** Appareil selon la revendication 2, dans lequel le premier événement électrique est une onde P, le second événement électrique est un signal électrique Y signalant une attaque de pression ventriculaire pendant une systole et l'intervalle de temps mesuré est un intervalle PY entre l'onde P et le signal électrique Y.

**19.** Appareil selon la revendication 18, dans lequel $AVD_c$ est calculé d'après une équation linéaire :

$$AVD_c = M1 \, (PY) - M2.$$

**20.** Appareil selon la revendication 19, dans lequel M1 est d'environ 0,96 et M2 est d'environ 139 millisecondes.

**21.** Appareil selon la revendication 19, dans lequel M1 est d'environ 1,0 et M2 est d'environ 150 millisecondes.

**22.** Appareil selon la revendication 18, dans lequel PY est mesuré en utilisant le générateur programmable d'impulsions ayant une électrode dans l'oreillette droite et le micromanomètre dans le ventricule gauche.

**23.** Appareil selon la revendication 22, dans lequel une onde P est détectée lorsqu'elle atteint 20 % d'une amplitude d'onde P maximale dans l'oreillette droite.

**24.** Appareil selon la revendication 18, dans lequel le signal électrique Y est produit par un phonogramme cardiaque.

**25.** Appareil selon la revendication 18, dans lequel le signal électrique Y est produit par un accéléromètre.

**26.** Appareil selon la revendication 18, dans lequel le signal électrique Y est produit par un enregistrement Doppler.

**27.** Appareil selon la revendication 18, dans lequel le signal électrique Y est produit par un dispositif de formation d'image d'écho.

**28.** Appareil selon la revendication 1, dans lequel le retard auriculo-ventriculaire souhaité comprend un retard auriculo-ventriculaire $AVD_{cs}$ qui produit un retard auriculo-ventriculaire approximativement optimal à la fois pour la contractilité et le volume systolique calculés par le générateur programmable d'impulsions à partir d'une application prédéterminée d'une relation de l'intervalle de temps mesuré à un retard auriculo-ventriculaire proche de l'optimal pour une variation de pression du ventricule gauche positive de pic maximal pendant une systole, et une relation d'une pression pulsatoire à des retards auriculo-ventriculaires normalisés.

**29.** Appareil selon la revendication 28, dans lequel le premier événement électrique est une onde P, le second événement électrique est un marqueur $R_m$ de détection ventriculaire droite, et l'intervalle de tempes mesuré est un intervalle $PR_m$ entre l'onde P et le marqueur $R_m$.

**30.** Appareil selon la revendication 29, dans lequel le retard auriculo-ventriculaire $AVD_{cs}$ est calculé d'après une équation linéaire :

$$AVD_{cs} = K3\ PR_m - K4\ \text{millisecondes.}$$

**31.** Appareil selon la revendication 30, dans lequel K3 est de 0,5 et K4 est de 15 millisecondes.

**32.** Appareil selon la revendication 30, dans lequel K3 est dans une plage de 0,4 à 0,6 et K4 est dans une plage de 0 à 30 millisecondes.

**33.** Appareil selon la revendication 1, dans lequel le retard auriculo-ventriculaire optimal comprend un retard auriculo-veritriculaire $AVD_s$ pour une pression pulsatoire maximale calculée par le générateur programmable d'impulsions à partir d'une application prédéterminée d'une relation de l'intervalle de temps mesuré à un retard auriculo-ventriculaire optimal pour une variation de pression du ventricule gauche positive, de pic maximal, pendant une systole afin de produire une approximation du retard auriculo-ventriculaire pour la stimulation du ventricule afin d'établir un volume systolique optimal.

**34.** Appareil selon la revendication 33, dans lequel le premier événement P est une onde P, le second événement électrique est un événement de pression pulsatoire x, et l'intervalle de temps mesuré est un intervalle PX entre l'onde P et l'événement de pression pulsatoire X.

**35.** Appareil selon la revendication 34, dans lequel le retard auriculo-ventriculaire $AVD_s$ est calculé d'après une équation linéaire :

$$AVD_s = K3\ PX - K4\ \text{millisecondes.}$$

**36.** Appareil selon la revendication 35, dans lequel K3 est de 1,22 et K4 est de 132 millisecondes.

**37.** Appareil selon la revendication 1, dans lequel le générateur programmable d'impulsions comprend un capteur d'événements électriques pouvant fonctionner de façon à contrôler un signal représentant une activité électrique ventriculaire, dans lequel le générateur programmable d'impulsions peut fonctionner pour déterminer un événement électrique ventriculaire Z qui a une relation de temps approximativement constante pour délivrer une stimulation ventriculaire qui maximise la variation de pression ventriculaire positive de pic, et pour délivrer une impulsion de stimulation en utilisant un temps de l'événement Z.

**38.** Appareil selon la revendication 37, dans lequel le signal représentant l'activité électrique ventriculaire comprend un électrogramme intracardiaque enregistré de façon endocardiaque ou épicardiaque à partir d'un ventricule.

**39.** Appareil selon la revendication 37, dans lequel le signal représentant l'activité électrique ventriculaire comprend un ECG de surface à 12 conducteurs.

**40.** Appareil selon la revendication 37, dans lequel l'événement est un début Q* d'un complexe QRS.

**41.** Appareil selon la revendication 37, dans lequel l'événement est un signal de marqueur délivré par un dispositif extérieur ou implantable.

**42.** Appareil selon la revendication 1, dans lequel le générateur programmable d'impulsions comporte un capteur d'événements électriques qui contrôle un signal représentant une activité mécanique auriculaire, dans lequel le générateur programmable d'impulsions détermine un événement mécanique auriculaire, X, qui une relation de temps constante avec le pic d'une systole auriculaire, et délivre une impulsion de stimulation en utilisant le temps d'un événement X.

**43.** Appareil selon la revendication 1, dans lequel le générateur programmable d'impulsions peut commuter entre :

la transmission d'impulsions de stimulation ventriculaire pour maximiser la contractilité en utilisant un retard auriculo-ventriculaire $AVD_c$ pour une variation de pression LV positive de pic maximal pendant une systole ;
la transmission d'impulsions de stimulation ventriculaire pour maximiser le volume systolique en utilisant un retard auriculo-ventriculaire $AVD_s$ pour une pression pulsatoire maximale ; et
la transmission d'impulsions de stimulation ventriculaire pour optimiser approximativement la contractilité et le volume systolique en utilisant un retard auriculo-ventriculaire $AVD_{cs}$ pour équilibrer une variation de pression LV positive de pic pendant une systole.

**44.** Appareil selon la revendication 43, dans lequel le retard auriculo-ventriculaire $AVD_c$ est calculé d'après une équation linéaire

$$AVD_c = K1\ (PQ^*) - K2,$$

dans laquelle PQ* représente un intervalle de temps mesuré entre une onde P et le commencement d'un complexe QRS, et
dans laquelle les constantes K1 et K2 sont dérivées de données échantillonnées provenant de plusieurs patients.

**45.** Appareil selon la revendication 43, dans lequel le retard auriculo-ventriculaire $AVD_c$ est calculé d'après une équation linéaire

$$AVD_c = N1\ (PR) - N2,$$

dans laquelle PR représente un intervalle de temps mesuré entre une onde P et une onde R, et dans laquelle les constantes N1 et N2 sont dérivées de données échantillonnées provenant de plusieurs patients.

**46.** Appareil selon la revendication 43, dans lequel le retard auriculo-ventriculaire $AVD_c$ est calculé d'après une équation linéaire

$$AVD_c = M1\ (PY) - M2,$$

dans laquelle PY représente un intervalle de temps mesuré entre une onde P et un départ de pression ventriculaire pendant une systole, et dans laquelle les constantes M1 et M2 sont dérivées de données échantillonnées provenant de plusieurs patients.

**47.** Appareil selon l'une quelconque des revendications 43 à 46, dans lequel le retard auriculo-ventriculaire $AVD_s$ est calculé d'après une équation linéaire

$$AVD_s = K3\,(PX) - k4,$$

dans laquelle PX représente un intervalle de temps mesuré entre une onde P et une pression pulsatoire, et dans laquelle les constantes K3 et K4 sont dérivées de données échantillonnées provenant de plusieurs patients.

48. Appareil selon l'une quelconque des revendications 43 à 47, dans lequel le retard auriculo-ventriculaire $AVD_{cs}$ est calculé d'après une équation linéaire

$$AVD_{cs} = K3\,(PR_m) - k4,$$

dans laquelle $PR_m$ représente un intervalle de temps mesuré entre une onde P et un marqueur de détection ventriculaire droite, et dans laquelle les constantes K3 et K4 sont dérivées de données échantillonnées provenant de plusieurs patients.

49. Appareil selon l'une quelconque des revendications 43 à 48, dans lequel le générateur programmable d'impulsions est conçu pour faire défaut au retard auriculo-ventriculaire $AVD_{cs}$ pour effectuer une commutation sur le retard auriculo-ventriculaire $AVD_c$ afin d'améliorer la contractilité et une commutation sur le retard auriculo-ventriculaire $AVD_s$ pour améliorer le volume systolique.

RIGHT SIDE | LEFT SIDE

SINOATRIAL NODE

ATRIAL MUSCLE

ATRIO-VENTRICULAR NODE

LA

RA

RV

LV

LEFT BUNDLE BRANCHES

RIGHT BUNDLE BRANCHES

VENTRICULAR MUSCLE

FIG. 1

RIGHT SIDE ; LEFT SIDE

FIG. 2

FIG. 3

FIG. 4A — LV INTRINSIC PRESSURE — CONTRACTILITY (NOT TO SCALE)

FIG. 4B — LV INTRINSIC ELECTROGRAM

FIG. 4C — OPTIMAL TIMING — RELATIVELY CONST FOR MAXIMUM CONTRACTILITY — ALL 3 COMBINED TO OPTIMIZE AVD$_C$ — TO OPTIMIZE

## FIG. 4D

PULSE PRESURE (NOT TO SCALE)

P

LV
INTRINSIC
PRESSURE

$P_I$

X

t

## FIG. 4E

OPTIMAL
TIMING

$AVD_S$

RELATIVELY CONSTANT FOR
PEAK PULSE PRESSURE

$P_P$

V

X

t

EP 1 079 893 B1

QRS → | SAMPLED QRS | → | DIGITAL FILTER 5pt, 2ms=T | → | PEAK DETECTION QRS | → | MAX SLOPE DETECTION OF Q-WAVE | → | 2% OF MAX SLOPE OF Q-WAVE | → Q*

**FIG. 5A**

EP 1 079 893 B1

FIG. 5B

FIG. 5C

EP 1 079 893 B1

FIG. 5D

FIG. 5E

FIG. 5F

EP 1 079 893 B1

FIG. 6

**350**

TEST PATIENT LV OUTPUT PRESSURE DURING PACING AT AV TIME DELAY INTERVAL

**360**

ANALYZE AV TIME DELAY INTERVALS AND LV OUTPUT PRESSURE TO DETERMINE AV TIME DELAY INTERVAL THAT PRODUCES MAXIMUM LV OUTPUT PRESSURE

**370**

GENERATE MODEL OF HEART FROM THE FEATURE TIME DIFFERENCE AND THE AV TIME DELAY INTERVAL

FIG. 7

FIG. 8A

FIG. 8B

FIG. 8C

FIG. 8D

FIG. 9

500

DETERMINE TIME INTERVAL FROM THE SENSED
ELECTROCARDIOGRAM SIGNALS

510

USE TIME INTERVAL IN MODEL TO GENERATE
ESTIMATED OPTIMAL AV TIME DELAY INTERVAL

520

PACE AT LEAST ONE VENTRICLE OF THE
HEART AT THE ESTIMATED OPTIMAL AV TIME
DELAY INTERVAL

FIG. 10

ACTUAL OPTIMAL DELAY vs PQ*

FIG. 11A

ACTUAL OPTIMAL DELAY vs PR

FIG. 11B

PREDICTION vs ACTUAL OPTIMAL DELAY
(USING PQ*, k1=0.93, k2=55.8)

y=0.9093x+8.0501
$R^2$=0.9123

PREDICTION(ms)

ACTUAL OPTIMAL AV DELAY (sm)

FIG. 12A

PREDICTION vs ACTUAL OPTIMAL DELAY
(USING PR, k3=0.82, k4=112.4)

y=0.8772x+12.397
$R^2$=0.8737

PREDICTION(ms)

ACTUAL OPTIMAL AV DELAY (sm)

FIG. 12B

FIG. 13